# EUROPEAN PATENT SPECIFICATION

(11) **EP 3 871 593 B1**
(45) Date of publication and mention of the grant of the patent: **15.05.2024**
(21) Application number: 19875167.9
(22) Date of filing: 25.10.2019
(51) Int. Cl.: A61B 5/02, A61B 5/0245, A61B 5/08, A61B 5/113, A61B 5/00, A61B 7/00, A61B 5/024, A61B 7/02, A61B 7/04

(54) **BIOLOGICAL SIGNAL MEASUREMENT DEVICE, BIOLOGICAL STATE INFERENCE DEVICE, AND BIOLOGICAL STATE INFERENCE SYSTEM**
VORRICHTUNG ZUR MESSUNG BIOLOGISCHER SIGNALE, INFERENZVORRICHTUNG FÜR BIOLOGISCHE ZUSTÄNDE UND INFERENZSYSTEM FÜR BIOLOGISCHE ZUSTÄNDE
DISPOSITIF DE MESURE DE SIGNAL BIOLOGIQUE, DISPOSITIF DE DÉDUCTION D'ÉTAT BIOLOGIQUE ET SYSTÈME DE DÉDUCTION D'ÉTAT BIOLOGIQUE

(30) Priority: 25.10.2018 JP 2018201347
(43) Date of publication of application: 01.09.2021
(73) Proprietor: Delta Tooling Co., Ltd., Hiroshima-shi, Hiroshima 736-0084 (JP)
(72) Inventor: FUJITA, Etsunori, Hiroshima-shi, Hiroshima 736-0084 (JP); OGURA, Yumi, Hiroshima-shi, Hiroshima 736-0084 (JP); TAKAICHI, Kanako, Aki-gun, Hiroshima 735-8501 (JP); MAEDA, Shinichiro, Aki-gun, Hiroshima 735-8501 (JP); HORIKAWA, Masahiro, Aki-gun, Hiroshima 735-8501 (JP); KANEKO, Shigehiko, Kawaguchi-shi, Saitama 333-0854 (JP); YOSHIZUMI, Masao, Hiroshima-shi, Hiroshima 730-0014 (JP)
(74) Representative: Hoffmann Eitle
(86) International application number: PCT/JP2019/042070
(87) International publication number: WO 2020/085512

(56) References cited:
- WO-A1-2017/099256
- WO-A1-2018/055782
- JP-A- 2010 094 466
- JP-A- 2010 284 517
- JP-A- 2012 024 390
- US-A- 5 159 935
- US-A- 5 432 755
- US-A- 5 853 005
- US-A1- 2011 060 215
- US-A1- 2013 060 149
- US-A1- 2016 367 213
- US-A1- 2017 156 632

## Description

### Technical Field

The present invention relates to a biological state inference system using a biological signal measurement device that captures, in a non-constraining manner, biological signals propagated through the dorsal body surface of a person and a biological state inference device that infers a state of the person by using time-series data of the biological signals captured by the biological signal measurement device.

### Background Art

In JP 2011-167362 A, JP 2014-117425 A, JP 2014-223271 A and JP 2016-26516 A and so on, the present inventors have proposed an art to capture, in a non-constraining manner, vibration generated on the dorsal body surface of the upper body of a person and infer a state of the person by analyzing the vibration. The vibration generated on the dorsal body surface of the upper body of a person is vibration propagated from a human body inner part such as the heart and the aorta and contains information on atrial and ventricular systoles and diastoles, information on vascular wall elasticity which serves as an auxiliary pump for circulation, and information on reflected waves.

In JP 2011-167362 A, slide calculation is performed in which a predetermined time width is set in a time-series waveform of a dorsal body surface pulse wave (Aortic Pulse Wave (APW)) of around 1 Hz extracted from vibration (biological signal) propagated through the body surface, to find a frequency gradient time-series waveform, and from the tendency of its variation, for example, based on whether its amplitude is on the increase or on the decrease, a biological state is estimated. It is also disclosed that, by frequency analysis of biological signals, power spectra of frequencies respectively corresponding to a function regulation signal, a fatigue reception signal, and an activity regulation signal that belong to a predetermined range from the ULF band (ultra-low-frequency band) to the VLF band (very-low-frequency band) are found, and a state of a person is determined from time-series variations of the respective power spectra.

JP 2014-117425 A and JP 2014-223271 A disclose a means for determining a homeostasis function level. For the determination, the means for determining the homeostasis function level uses at least one or more of plus/minus of a differentiated waveform of a frequency gradient time-series waveform, plus/minus of an integrated waveform obtained by integrating the frequency gradient time-series waveform, absolute values of frequency gradient time-series waveforms obtained by absolute value processing of a frequency gradient time-series waveform found by a zero-cross method and a frequency gradient time-series waveform found by a peak detection method, and so on. By using the combination of these, it is found on which level the homeostasis function is. Further, JP 2016-26516 A discloses a sound/vibration information collection mechanism including a resonance layer including a natural oscillator having a natural frequency corresponding to sound/vibration information of a biological signal.

A biological state inference system is also disclosed is also known from US 5 853 005 A forming the basis for the preamble of claim 1.

### Disclosure of the Invention

### Problems to Be Solved by the Invention

In all the devices for collecting biological signals disclosed in JP 2011-167362 A, JP 2014-117425 A, JP 2014-223271 A and JP 2016-26516 A, in a base member made of a plate-shaped bead foam, placement holes are formed on the left and right of a position corresponding to the backbone, three-dimensional knitted fabrics are fit in the placement holes, films cover their both surfaces to make the placement holes airtight spaces, and the three-dimensional knitted fabrics are supported therein. However, an acoustic sensor that captures vibration (acoustic wave information) transmitted from the body surface to the three-dimensional knitted fabrics obtains information on left cardiac acoustic waves including apex beats and accordingly is disposed in the left placement hole. Therefore, it is practically the three-dimensional knitted fabric disposed in the left placement hole and the acoustic sensor that function as a biological signal detection unit, and the three-dimensional knitted fabric placed in the right placement hole only functions mainly to keep the left and right balance when supporting the back.

Further, the biological state inference devices of JP 2011-167362 A, JP 2014-117425 A, JP 2014-223271 A and JP 2016-26516 A are proposed for use mainly to estimate a state of an automobile driver through the determination of a hypnagogic symptom signal, the estimation of fatigue, and so on, to inhibit the driver's drowsy driving or stimulate the driver into an awakening state. However, the means by the present inventors that uses the base member made of the bead foam and the films, houses the three-dimensional knitted fabrics in the closed placement holes which are insulated from the outside, makes the three-dimensional knitted fabrics function as the natural oscillators, and uses the acoustic sensor to obtain, as the acoustic wave information, the biological signal propagated through the body surface is expected to be applied not only to the detection of a doze but also to medical fields such as medical checkups and the like, as a tool to obtain a variety of information of a living body.

The present invention was made in consideration of the above and has an object to provide a biological signal measurement device capable of obtaining a variety of biological information and applicable also to medical fields and the like, a biological state inference device capable of appropriately inferring a target biological state by using time-series data of biological signals obtained from the biological signal device, and a biological state inference system using these.

### Means for Solving the Problems

To solve the above problem, a biological state interference system as defined in claim 1 is disclosed.

Preferably, the filtering frequency deciding means is a means which decides a filtering frequency for respiratory physiology information for use in filtering into time-series data mainly containing respiratory physiology information, by using two frequency analysis results of the time-series data of the biological signal from the left upper part biological signal detection unit and the time-series data of the biological signal from the lower part biological signal detection unit, and
the inference-use processed waveform calculating means applies the filtering frequency for respiratory physiology information to the time-series data of the biological signal obtained from at least one of the left upper part biological signal detection unit, the right upper part biological signal detection unit, and the lower part biological signal detection unit and performs the arithmetic processing to obtain a pseudo-respiratory waveform as the inference-use processed waveform.

As the inferring means, a means which compares data of the two or more pseudo-respiratory waveforms to evaluate activity of a respiratory muscle can be provided.

Preferably, the filtering frequency deciding means is a means which decides a filtering frequency for heart sound information for use in filtering into time-series data mainly containing heart sound information, by using two frequency analysis results of the time-series data of the biological signal from the left upper part biological signal detection unit and the time-series data of the biological signal from the lower part biological signal detection unit,
the inference-use processed waveform calculating means applies the filtering frequency for heart sound information to the time-series data of the biological signal obtained from at least one of the left upper part biological signal detection unit, the right upper part biological signal detection unit, and the lower part biological signal detection unit and performs the arithmetic processing to obtain a pseudo-heart sound waveform as the inference-use processed waveform.

Preferably, after the filtering processing, auralization processing is performed to generate the pseudo-heart sound waveform.

Preferably, the auralization processing is clipping processing or heterodyne processing.

Preferably, the inferring means includes a means which finds a time lag between the pseudo-heart sound waveform and heart sound data obtained from a phonocardiograph, creates a Lorenz plot by using the time lag, and infers the biological state from a variance state in the Lorenz plot.

### Effect of the Invention

The biological signal measurement device of the biological state inference system of the present invention includes the three biological signal detection units, namely, the left upper part biological signal detection unit which obtains the time-series data of the biological signal containing the central circulatory system information and the peripheral circulatory system information that are mainly related to the activity of the left cardiac system and the respiratory physiology information that is mainly related to the activity of the left lung; the right upper part biological signal detection unit which obtains the time-series data of the biological signal containing the respiratory physiology information mainly related to the activity of the right lung; and the lower part biological signal detection unit which obtains the time-series data of the biological signal containing: the abdominal respiratory physiology information mainly related to the activities of the left lung and the right lung and transmitted through the diaphragm; and peripheral circulatory system information. Therefore, the use of an appropriate combination of the time-series data obtained from the three biological signal detection units enables the biological state inference device to obtain the highly precise inference-use processed waveform from which electrical noise has been removed. Further, because the precision of the inference-use processed waveform corresponding to target biological information on breathing, heart sound, or the like increases, the precision of inferring the biological state also increases. Therefore, the device is suitable for application to medical fields such as medical checkups.

### Brief Description of the Drawings

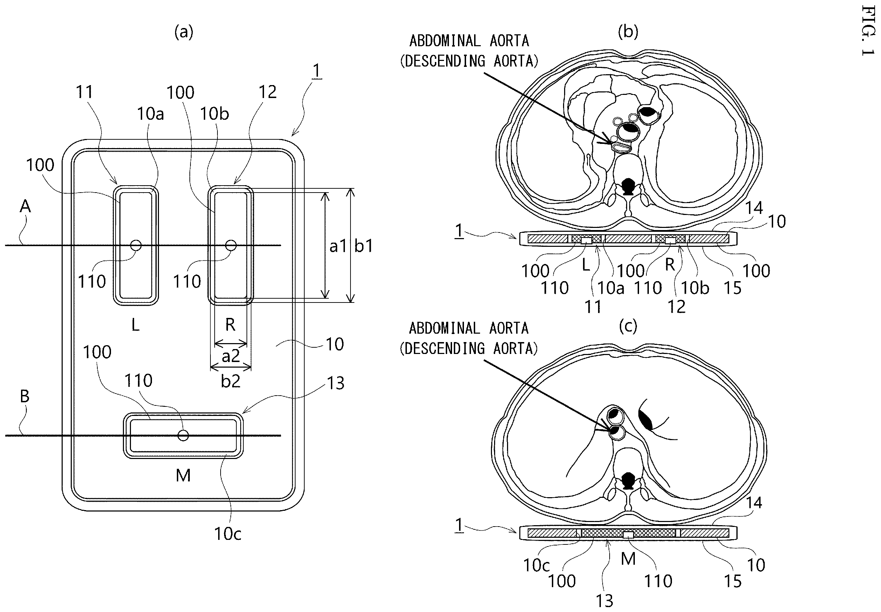

[FIGs. 1] FIG. 1(a) is a plan view illustrating a biological signal measurement device according to one embodiment of the present invention, FIG. 1(b) is a horizontal sectional view illustrating arrangement positions of a left upper part biological signal detection unit (L) and a right upper part biological signal detection unit (R) in relation to the body of a person when the biological signal measurement device is disposed on the back side of the person, and FIG. 1(c) is a horizontal sectional view illustrating an arrangement position of a lower part biological signal detection unit (M) in relation to the body of the person.
[FIG. 2] FIG. 2 is a vertical sectional view illustrating the arrangement positions of the left upper part biological signal detection unit (L), the right upper part biological signal detection unit (R), and the lower part biological signal detection unit (M) in relation to the body of the person when the biological signal measurement device is disposed on the back side of the person.
[FIG. 3] FIG. 3 is an enlarged sectional view illustrating an essential part of the biological signal measurement device.
[FIG. 4] FIG. 4 is an explanatory block diagram of the configuration of a biological state inference device.
[FIG. 5] is a flowchart illustrating processes in which the biological state inference device infers biological states by using time-series data transmitted from acoustic sensors of the biological signal measurement device.
[FIGs. 6] FIG. 6(a) to (c) are data of a subject H, out of which (a) is a chart illustrating the result of a frequency analysis of time-series data from the acoustic sensor (L) of the left upper part biological signal detection unit, (b) is a chart illustrating the result of a frequency analysis of time-series data from the acoustic sensor (M) of the lower part biological signal detection unit, and (c) is a chart illustrating the result of finding a ratio (L/M) of power spectra of these.
[FIGs. 7] FIGs. 7(a) to (c) are data of a subject M, out of which (a) is a chart illustrating the result of a frequency analysis of time-series data from the acoustic sensor (L) of the left upper part biological signal detection unit, (b) is a chart illustrating the result of a frequency analysis of time-series data from the acoustic sensor (M) of the lower part biological signal detection unit, and (c) is a chart illustrating the result of finding a ratio (L/M) of power spectra of these.
[FIGs. 8] FIGs. 8(a) to (c) are data of a subject N, out of which (a) is a chart illustrating the result of a frequency analysis of time-series data from the acoustic sensor (L) of the left upper part biological signal detection unit, (b) is a chart illustrating the result of a frequency analysis of time-series data from the acoustic sensor (M) of the lower part biological signal detection unit, and (c) is a chart illustrating the result of finding a ratio (L/M) of power spectra of these.
[FIGs. 9] FIG. 9(a) is a chart illustrating pseudo-respiratory waveforms of the subject H, and FIG. 9(b) is a chart illustrating a corresponding output waveform of a breathing sensor.
[FIGs. 10] FIG. 10(a) is a chart illustrating pseudo-respiratory waveforms of the subject M, and FIG. 10(b) is a chart illustrating a corresponding output waveform of the breathing sensor.
[FIGs. 11] FIG. 11(a) is a chart illustrating pseudo-respiratory waveforms of the subject N, and FIG. 11(b) is a chart illustrating a corresponding output waveform of the breathing sensor.
[FIGs. 12] FIGs. 12(a), (b) are charts illustrating waveforms of electrocardiograms (top), waveforms of heart sound (middle), and pseudo-heart sound waveforms (bottom) of the subject N in an initial breathlessness period and a latter breathlessness period respectively.
[FIGs. 13] FIGs. 13(a), (b) are charts illustrating waveforms of electrocardiograms (top), waveforms of heart sound (middle), and pseudo-heart sound waveforms (bottom) of the subject N in an initial effort breathing period and a latter effort breathing period respectively.
[FIGs. 14] FIGs. 14(a), (b) are charts illustrating waveforms of electrocardiograms (top), waveforms of heart sound (middle), and pseudo-heart sound waveforms (bottom) of the subject N in an initial natural breathing period and a latter natural breathing period respectively.
[FIGs. 15] FIGs. 15(a), (b) are explanatory charts of time lags between heart sound data from a phonocardiograph and data of pseudo-heart sound.
[FIGs. 16] FIGs. 16(a) to (c) are data of the subject H, (a) being a chart illustrating a time-series waveform found by L/(M × R), (b) being a chart illustrating a pseudo-heart sound waveform resulting from auralization by heterodyne processing, and (c) being a chart illustrating heart sound data from a phonocardiograph at the same timing.
[FIGs. 17] FIGs. 17(a) to (c) are data of the subject M, (a) being a chart illustrating a time-series waveform found by L/(M × R), (b) being a chart illustrating a pseudo-heart sound waveform resulting from auralization by heterodyne processing, and (c) being a chart illustrating heart sound data from a phonocardiograph at the same timing.
[FIGs. 18] FIGs. 18(a) to (c) are data of the subject N, (a) being a chart illustrating a time-series waveform found by L/(M × R), (b) being a chart illustrating a pseudo-heart sound waveform resulting from auralization by heterodyne processing, and (c) being a chart illustrating heart sound data from a phonocardiograph at the same timing.
[FIGs. 19] FIGs. 19(a) to (c) are data of another subject, (a) being a chart illustrating data of an electrocardiogram, (b) being a chart illustrating data from a phonocardiograph, (c) being a chart illustrating a filtered waveform for a pseudo-heart sound waveform, (d) being a chart illustrating a waveform resulting from auralization by heterodyne processing, and (e) being a chart illustrating a waveform resulting from clipping processing.
[FIGs. 20] FIGs. 20(a) to (c) are charts illustrating relations between an R wave time interval (RRI) of a phonocardiogram and a first-sound time interval of a pseudo-heart sound waveform (I-sound interval of pseudo-heart sound).
[FIGs. 21] FIGs. 21(a) to (e) are charts illustrating waveforms in a process of processing time-series data from the acoustic sensor (R) of the right upper part biological signal detection unit during the effort breathing of the subject N to obtain a pseudo-respiratory waveform.
[FIGs. 22] FIGs. 22(a) to (e) are charts illustrating waveforms in a process of processing time-series data from the acoustic sensor (R) of the right upper part biological signal detection unit during the natural breathing of the subject N to obtain a pseudo-respiratory waveform.
[FIGs. 23] FIGs. 23(a) to (e) are charts illustrating waveforms in a process of processing time-series data from the acoustic sensor (R) of the right upper part biological signal detection unit during the effort breathing of the subject H to obtain a pseudo-respiratory waveform.
[FIGs. 24] FIGs. 24(a) to (e) are charts illustrating waveforms in a process of processing time-series data from the acoustic sensor (R) of the right upper part biological signal detection unit during the natural breathing of the subject H to obtain a pseudo-respiratory waveform.

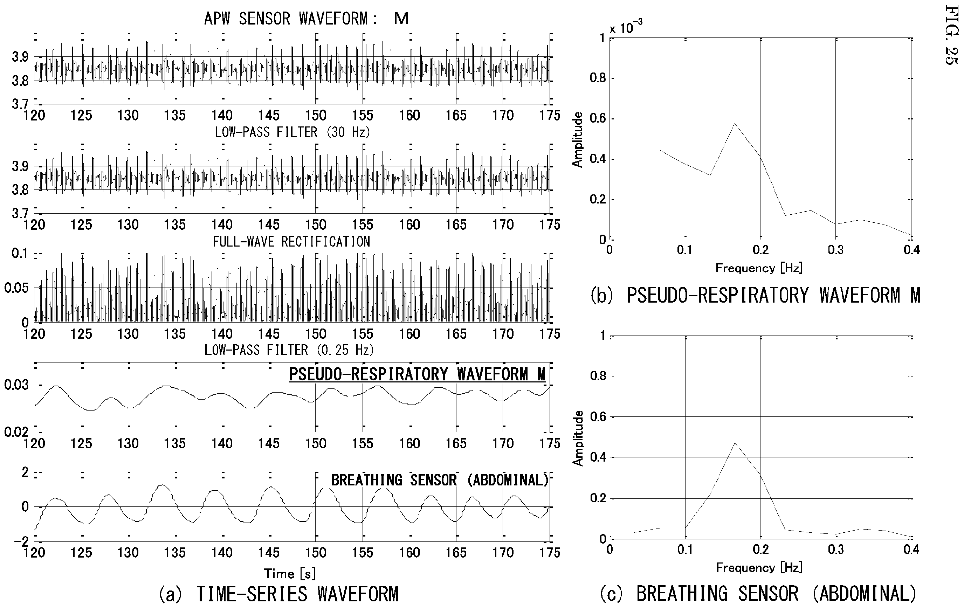

[FIGs. 25] FIGs. 25(a) is a chart illustrating, in order from the top, time-series data from the acoustic sensor (M) of the lower part biological signal detection unit in a time zone of natural breathing, a filtered waveform for a pseudo-respiratory waveform, a waveform resulting from full-wave rectification, a pseudo-respiratory waveform, and a waveform of the breathing sensor, FIG. 25(b) is a chart illustrating the result of a frequency analysis of the pseudo-respiratory waveform, and FIG. 25(c) is a chart illustrating the result of a frequency analysis of the waveform of the breathing sensor.
[FIGs. 26] FIGs. 26(a) to (d) are explanatory charts of a process of finding a Mayer wave using data that is measured from the subject N in a supine posture.
[FIGs. 27] FIG. 27(a) is a chart illustrating another example of finding a time-series waveform of a Mayer wave using time-series data from the acoustic sensor (M) of the lower part biological signal detection unit, FIG. 27(b) is a chart illustrating the result of a frequency analysis thereof, and FIG. 27(c) is a chart illustrating the result of a frequency analysis of a finger plethysmogram.

### Description of Embodiments

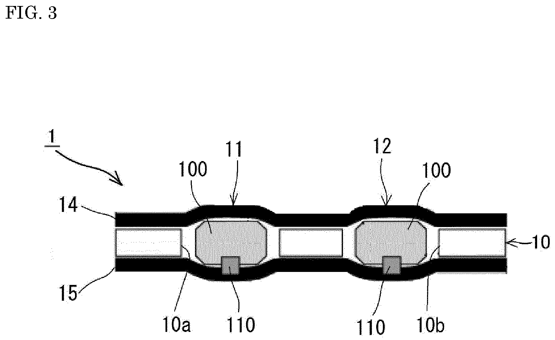

The present invention will be hereinafter described in more detail based on an embodiment of the present invention illustrated in the drawings. As illustrated in FIGs. 1 to FIG. 3, in a biological signal measurement device 1 according to this embodiment, three biological signal detection units, namely, a left upper part biological signal detection unit 11, a right upper part biological signal detection unit 12, and a lower part biological signal detection unit 13 are provided in a base member 10.

The base member 10 is made of a plate-shaped body having an area large enough to include the three biological signal detection units 11 to 13 and cover a range from the chest to the abdomen of a person. It is preferably formed of a material such as a flexible synthetic resin that gives only a small uncomfortable feeling when the back of the person abuts thereon and is more preferably formed of a bead foam. Thin films of beads forming the bead foam vibrate by sensitively responding to body surface microvibration that is based on biological signals to easily propagate the biological signals to the biological signal detection units 11 to 13.

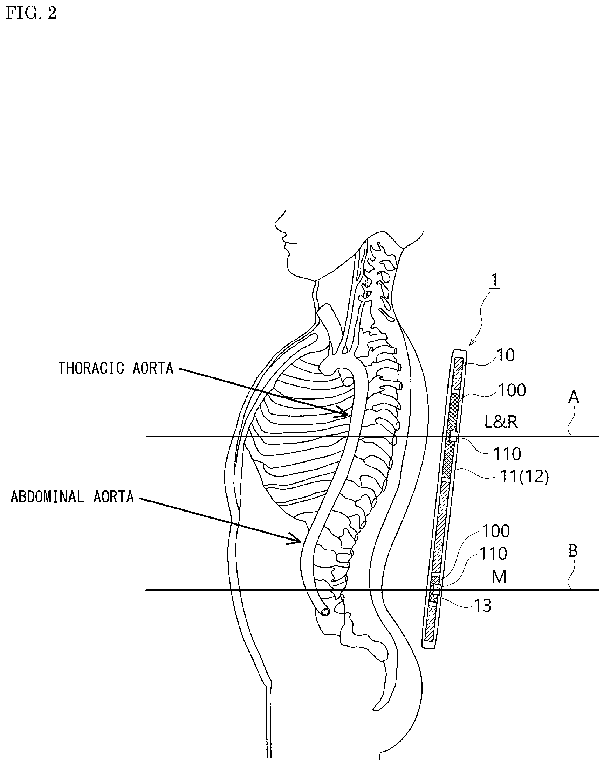

In the state in which the base member 10 is disposed along the back of the person, above (on the shoulder side of) a diaphragm-corresponding position corresponding to the position of the diaphragm of the person, two detection unit placement holes 10a, 10b are formed at a position corresponding to the position of the heart (near the line indicated by reference sign A in FIG. 1 and FIG. 2), and under (on the waist side of) the diaphragm-corresponding position, one detection unit placement hole 10c is formed at a position corresponding to the position of the waist (near the line indicated by reference sign B in FIG. 1 and FIG. 2). The two detection unit placement holes 10a, 10b on the upper side are provided at a predetermined interval on the left and right of a backbone-corresponding position corresponding to the position of the backbone of the person. Further, the two detection unit placement holes 10a, 10b on the upper side are substantially in a vertically-long rectangular shape that is longer in the up-down direction, and the detection unit placement hole 10c on the lower side is substantially in a laterally-long rectangular shape that is longer in the left-right direction. To enable the capturing of biological signals of a range corresponding to the lungs and the heart, the two detection unit placement holes 10a, 10b on the upper side are vertically long, and to enable the capturing of abdominal information that is based on the activity of the left and right lungs and is transmitted through the diaphragm, the detection unit placement hole 10c on the lower side is laterally long. This corresponds to the shapes and the arrangement directions of the biological signal detection units 11 to 13.

The biological signal detection units 11 to 13 each have a three-dimensional knitted fabric 100 and an acoustic sensor 110 constituted by a microphone. The three-dimensional knitted fabric 100 is formed of a pair of ground knitted fabrics disposed apart from each other and connecting yarns connecting the ground knitted fabrics as is disclosed in the aforesaid JP 2011-167362 A or JP 2002-331603 A proposed by the present inventors. For example, the ground knitted fabrics each can be formed to have a flat knitted fabric structure (fine meshes) continuous both in a wale direction and a course direction using yarns of twisted fibers or to have a knitted fabric structure having honeycomb (hexagonal) meshes. The connecting yarns impart predetermined rigidity to the three-dimensional knitted fabric so that one of the ground knitted fabrics and the other ground knitted fabric are kept at a predetermined interval. Therefore, applying tension in a planar direction makes it possible to cause string vibration of the yarns of the facing ground knitted fabrics forming the three-dimensional knitted fabric or of the connecting yarns connecting the facing ground knitted fabrics. Accordingly, cardio-vascular sound/vibration being a biological signal causes the string vibration and is propagated in the planar direction of the three-dimensional knitted fabric.

As a material of the yarns forming the ground knitted fabrics or the connecting yarns of the three-dimensional knitted fabric, various materials are usable, and examples thereof include synthetic fibers and regenerated fibers such as polypropylene, polyester, polyamide, polyacrylonitrile, and rayon, and natural fibers such as wool, silk, and cotton. These materials each may be used alone or any combination of these may be used. Preferably, the material is a thermoplastic polyester-based fiber represented by polyethylene terephthalate (PET), polybutylene terephthalate (PBT), and the like, a polyamide-based fiber represented by nylon 6, nylon 66, and the like, a polyolefin-based fiber represented by polyethylene, polypropylene, and the like, or a combination of two kinds or more of these fibers. Further, the shape of the ground yarns or the connecting yarns is not limited either, and a round cross-section yarn, a modified cross-section yarn, a hollow yarn, or the like may be used. Further, a carbon yarn, a metallic yarn, or the like is also usable.

### Examples of the usable three-dimensional knitted fabric are as follows.

(a) Product number: 49013D (manufactured by Suminoe Textile Co., Ltd.), thickness 10 mm
   Material:
   Front-side ground knitted fabric ... twisted yarn of two false twist yarns of 450 decitex/108f polyethylene terephthalate fibers
   Rear-side ground knitted fabric ... twisted yarn of two false twist yarns of 450 decitex/108f polyethylene terephthalate fibers
   Connecting yarns ... 350 decitex/1f polytrimethylene terephthalate monofilament
(b) Product No.: AKE70042 (manufactured by Asahi Kasei Corporation), thickness 7 mm
(c) Product No.: T28019C8G (manufactured by Asahi Kasei Corporation), thickness 7 mm

The three-dimensional knitted fabrics 100 forming the biological signal detection units 1 1 to 13 are formed in a substantially rectangular shape corresponding to the aforesaid detection unit placement holes 10a to 10c. Then, films 14, 15 are stacked on both surfaces of the base member 10 to cover the front surfaces and the rear surfaces of the three-dimensional knitted fabrics 100. The films 14, 15 each may have a size corresponding to each of the detection unit placement holes 10a to 10c, or the films 14, 15 each may have a size that can cover, by itself, all the three detection unit placement holes 10a to 10c. Consequently, the detection unit placement holes 10a to 10c become resonance boxes to have a function of amplifying weak biological signals.

The three-dimensional knitted fabrics 100 preferably have a thickness large enough to be higher than the detection unit placement holes 10a to 10c when they are placed in the detection unit placement holes 10a to 10c. When the films 14, 15 are stacked on the surfaces of the base member 10, the films 14, 15 cover both the front surfaces and the rear surfaces of the three-dimensional knitted fabrics 100, and at this time, the use of the three-dimensional knitted fabrics 100 having a larger thickness than the thickness of the base member 10 corresponding to the depth of the detection unit placement holes 10a to 10c results in an increase in tension of the three-dimensional knitted fabrics 100 when they are sandwiched by the films 14, 15 because they are supported in the detection unit placement holes 10a to 10c while pressed by the films 14, 15, so that the string vibration of the yarns forming the three-dimensional knitted fabrics 100 more easily occurs in the detection unit placement holes 10a to 10c functioning as the resonance boxes.

Preferably, in each of the three-dimensional knitted fabrics 100, the outer peripheral length and width (a1, a2) which are dimensions along its outer periphery are shorter than the inner peripheral length and width (b1, b2) which are dimensions along the inner periphery of each of the detection unit placement holes 10a to 10c (see FIG. 1), and even with such dimensions, the three-dimensional knitted fabrics 100 are supported with little displacement in the detection unit placement holes 10a to 10c since they are pressed by the films 14, 15 from both surfaces. As a result of forming the three-dimensional knitted fabrics 100 such that the outer peripheral length and width (a1, a2) which are the dimensions along their outer peripheries are shorter than the inner peripheral length and width (b1, b2) of the detection unit placement holes 10a to 10c, the outer peripheries of the three-dimensional knitted fabrics 100 are apart from the inner peripheries. A gap therebetween is preferably within a range of 1 to 10 mm, which enhances the resonance operation in the detection unit placement holes 10a to 10c to more easily cause the string vibration of the three-dimensional knitted fabrics 100, enabling the acoustic sensors to more easily collect the vibration (sound) generated by the biological signals.

As described above, the left upper part biological signal detection unit 11 is disposed at the position that is above the diaphragm-corresponding position and is on the left side of the backbone-corresponding position and accordingly obtains time-series data of a biological signal containing central circulatory system information and peripheral circulatory system information that are mainly related to the activity of the left cardiac system and respiratory physiology information that is mainly related to the activity of the left lung.

The right upper part biological signal detection unit 12 is disposed at the position that is above the diaphragm-corresponding position and is on the right side of the backbone-corresponding position and accordingly obtains time-series data of a biological signal containing respiratory physiology information mainly related to the activity of the right lung.

The lower part biological signal detection unit 13 disposed under the diaphragm-corresponding position obtains time-series data of a biological signal containing: abdominal respiratory physiology information mainly related to the activities of the left lung and the right lung and transmitted through the diaphragm; and peripheral circulatory system information.

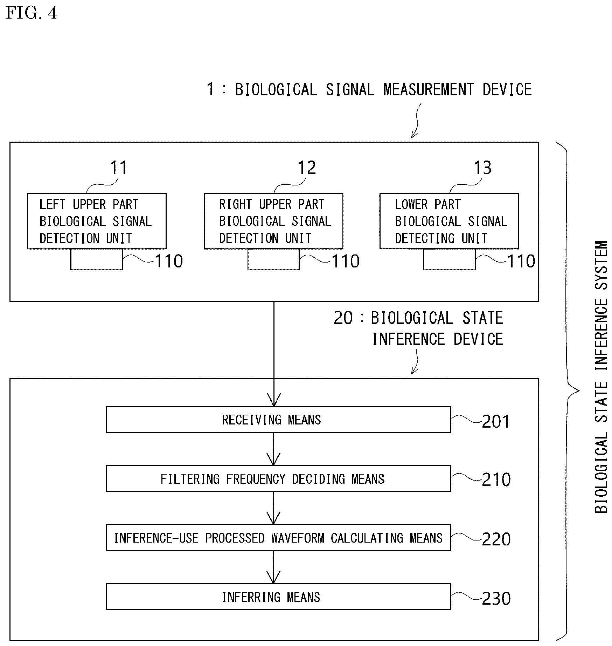

Next, a biological state inference device 20 with a computer function in which a computer program for processing data obtained from the biological signal measurement device 1 of this embodiment is set will be described. Note that a combination of the biological signal measurement device 1 and the biological state inference device 20 is a biological state inference system specified in the claims (see FIG. 4).

The biological state inference device 20 infers biological states by processing the time-series data of the biological signals obtained by the biological signal detection units 11 to 13 of the biological signal measurement device 1. The biological state inference device 20 is constituted by a computer (including a personal computer, a microcomputer incorporated in a device, and the like) and receives the time-series data of the biological signals transmitted from the acoustic sensors 110 of the biological signal measurement device 1. It includes a filtering frequency deciding means 210, an inference-use processed waveform calculating means 220, and an inferring means 230 which perform predetermined processing using the received time-series data.

The biological state inference device 20 is provided with a computer program that causes the execution of procedures functioning as the filtering frequency deciding means 210, the inference-use processed waveform calculating means 220, and the inferring means 230 and that is stored in a storage unit (including not only a recording medium such as a hard disk built in the computer (biological state inference device 20) but also any of various removable recording media and a recording medium of another computer connected through a communication means). Further, it functions as the filtering frequency deciding means 210, the inference-use processed waveform calculating means 220, and the inferring means 230 as the computer program to cause the computer to execute the procedures. Further, the biological state inference device 20 can be implemented by an electronic circuit having one storage circuit or more in which the computer program implementing the filtering frequency deciding means, the inference-use processed waveform calculating means 220, and the inferring means 230 is incorporated.

Further, the computer program can be provided in a state of being stored in a recording medium. The recording medium storing the computer program may be a non-transitory recording medium. The non-transitory recording medium is not limited, and examples thereof are recording media such as a flexible disk, a hard disk, CD-ROM, MO (magneto-optical disk), DVD-ROM, and a memory card. Further, the computer program can be transmitted to the computer through a communication line to be installed therein.

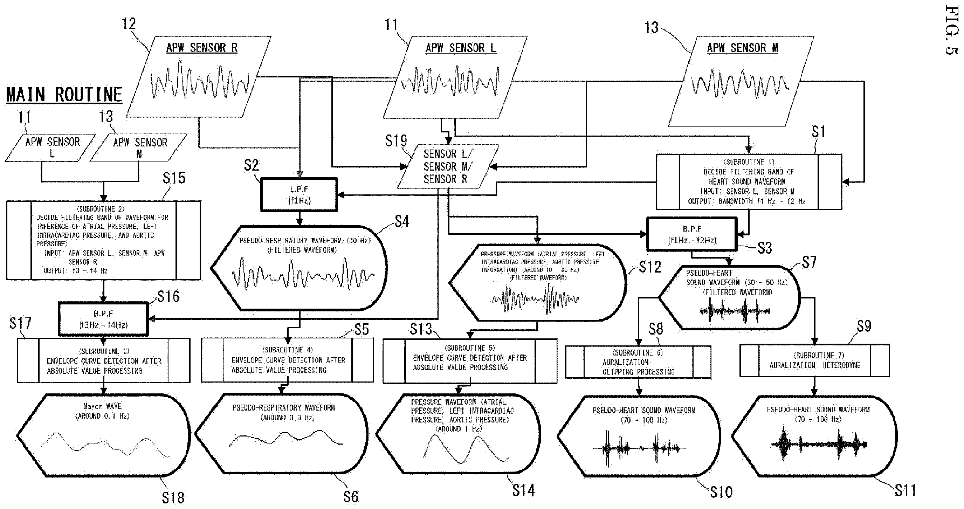

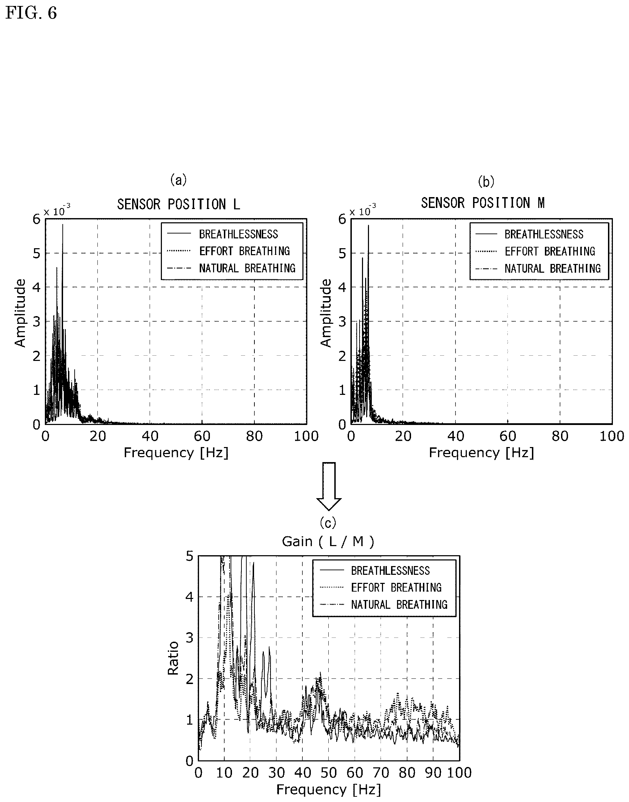

The filtering frequency deciding means 210 decides a filtering frequency for use in filtering the time-series data of the biological signals which are transmitted from the acoustic sensors 110 assembled in the biological signal detection units 11 to 13 of the biological signal measurement device 1 and received by a receiving means 201. For deciding the filtering frequency, two or three of the time-series data of the biological signals transmitted from the biological signal detection units 11 to 13 are used, which makes it possible to erase noise and facilitate deciding the filtering frequency for each individual, leading to improved precision of the inference of the biological state. This embodiment is configured to decide the filtering frequency using the time-series data of the biological signals obtained from the acoustic sensors 110 of the left upper part biological signal detection unit 11 and the lower part biological signal detection unit 13 (S1 in FIG. 5). Specifically, the time-series data of the biological signals obtained from the acoustic sensors 110 of the left upper part biological signal detection unit 11 and the lower part biological signal detection unit 13 are frequency-analyzed and a ratio between obtained power spectra corresponding to each frequency is found, and according to the result, the filtering frequency is decided (see FIGs. 6 to FIGs. 8). Owing to the use of the ratio therebetween, electrical noise is erased. Further, since the values measured by the acoustic sensors 110 of the left upper part biological signal detection unit 11 and the lower part biological signal detection unit 13 are used as illustrated in FIGs. 6 to FIGs. 8, it is possible to find an appropriate filtering frequency for each subject of the measurement.

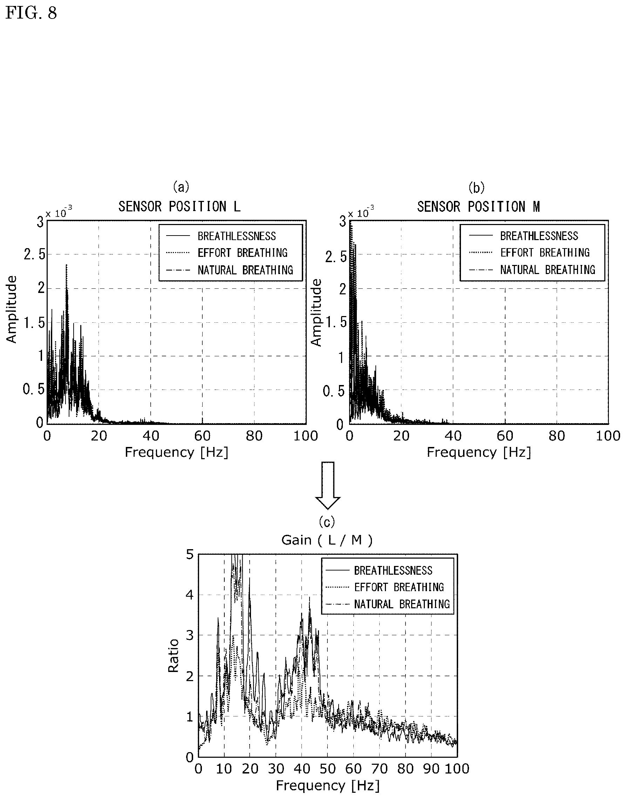

For example, in the example in FIGs. 8, based on an amplitude change of the ratio on the vertical axis, 30 Hz and 50 Hz are selected as the filtering frequencies. Then, for the filtering into time-series data mainly containing respiratory physiology information, a low-pass filter (L.P.F.) whose cutoff frequency is 30 Hz is set (S2 in FIG. 5), and for the filtering into time-series data mainly containing heart sound information, a band-pass filter (B.P.F.) whose pass frequency band is 30 to 50 Hz is set (S3 in FIG. 5).

The inference-use processed waveform calculating means 220 filters the time-series data of the biological signal obtained from each of the acoustic sensors 110 assembled in the biological signal detection units 11 to 13, using the filtering frequency decided by the filtering frequency deciding means 210. Thereafter, it executes necessary arithmetic processing to find an inference-use processed waveform.

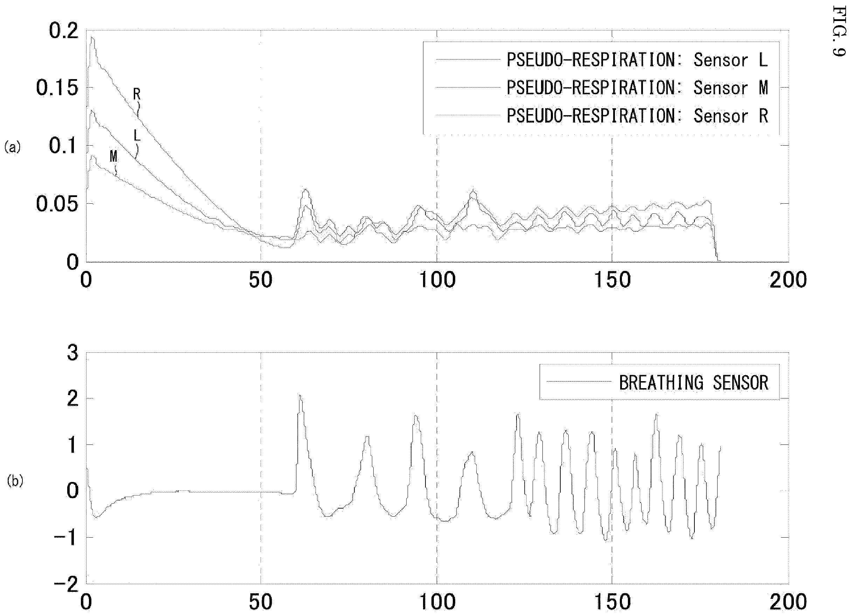

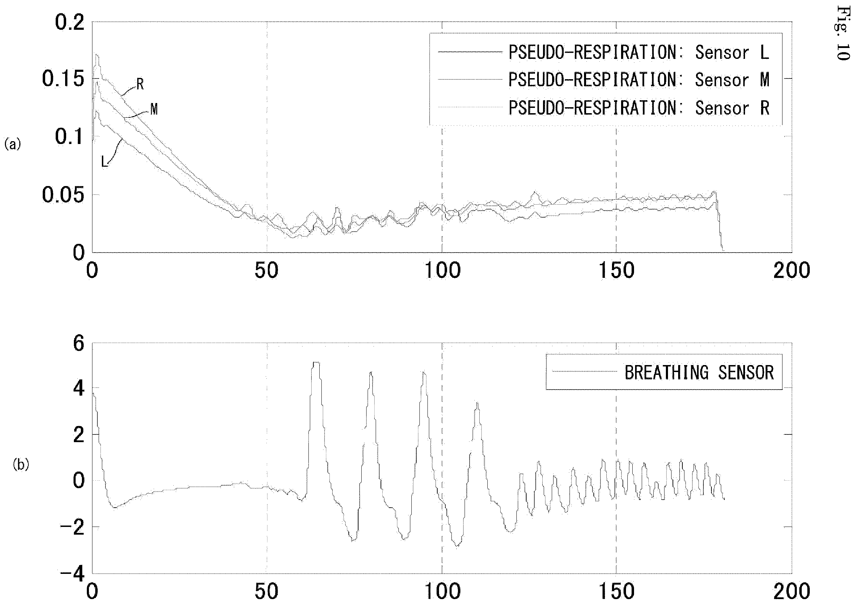

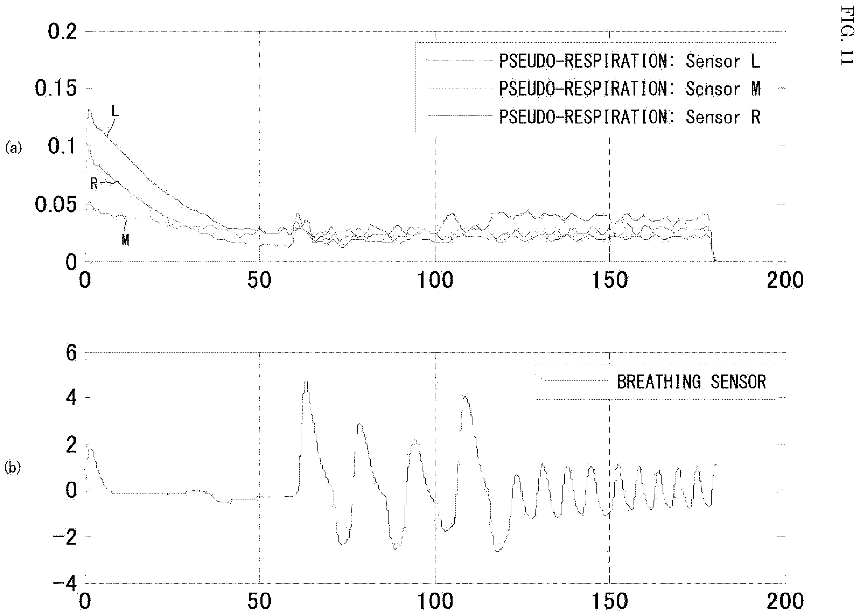

In the case where the aforesaid 30 Hz low-pass filter is used (S2 in FIG. 5), a filtered waveform for use in obtaining a final pseudo-respiratory waveform is obtained (S4 in FIG. 5). This filtered waveform is further subjected to necessary arithmetic processing, in this embodiment, absolute value processing, and a waveform resulting from the absolute value processing is subjected to detection processing and is further demodulated by finding an envelope curve connecting its peak values, whereby a pseudo-respiratory waveform of around 0.3 Hz is found as the inference-use processed waveform (S5 and S6 in FIG. 5). The time-series data from the three acoustic sensors 110 provided in the left upper part biological signal detection unit 11, the right upper part biological signal detection unit 12, and the lower part biological signal detection unit 13 respectively are preferably used to find the pseudo-respiratory waveforms reflecting the respiratory physiology information. The time-series data from the left upper part biological signal detection unit 11, the right upper part biological signal detection unit 12, and the lower part biological signal detection unit 13 all include the respiratory physiology information. Therefore, by studying a difference among the pseudo-respiratory waveforms obtained from the time-series data from the three acoustic sensors 110, the later-described inferring means 230 is capable of finding a breathing state, for example, which of thoracic breathing or abdominal breathing is predominant, how the respiratory muscles are acting, and so on (see FIGs. 9 to FIGs. 11).

In the case where the 30 to 50 Hz band-pass filter is applied (S3 in FIG. 5) to each of the time-series waveforms found at S19 in FIG. 5, a filtered waveform for a pseudo-heart sound waveform is obtained (S7 in FIG. 5), details of which will be described later. Further applying auralization processing (S8, S9 in FIG. 5) to this filtered waveform results in a pseudo-heart sound waveform of 70 to 100 Hz (S10, S 11 in FIG. 5). The auralization processing can be clipping processing (S8 in FIG. 5) or heterodyne processing (S9 in FIG. 5), for instance. In order to reduce the influence of the respiratory physiology information in the pseudo-heart sound waveform, it is preferable to divide the time-series data obtained from the acoustic sensor 110 of the left upper part biological signal detection unit 11, which data contains more information on apex beats, by the time-series data obtained from the acoustic sensors 110 of the right upper part biological signal detection unit 12 and the lower part biological signal detection unit 13 to find time-series data mainly regarding the apex beats, and apply the aforesaid 30 to 50 Hz band-pass filter to the found time-series data.

Further, the aforesaid time-series data mainly regarding the apex beats includes less respiratory physiology information and contains many pieces of information on not only the apex beats but also left atrial pressure, left intracardiac pressure, and aortic pressure. Therefore, by applying a 10 to 30 Hz band-pass filter thereto, the inference-use processed waveform calculating means 220 is capable of finding a filtered waveform for a pseudo-waveform of an aortic pulse wave (APW) (S12 in FIG. 5) as is disclosed in JP 2016-26516 A by the present inventors. This filtered waveform is a carrier wave including a low-frequency vibration waveform reflecting the autonomic nervous function as is disclosed in JP 2016-26516 A, and therefore, after it is subjected to absolute value processing, a detector circuit performs the full-wave rectification of the resultant and demodulates it by finding an envelope curve connecting its peak values, and APW of around 1 Hz which is a low-frequency biological signal is extracted (S13, S14 in FIG. 5).

Further, a band-pass filter whose filtering frequency is decided using the time-series data of the biological signals obtained from the acoustic sensors 110 of the left upper part biological signal detection unit 11 and the lower part biological signal detection unit 13, for example, a 30 to 50 Hz band-pass filter or a 50 to 70 Hz band-pass filter, is applied to the aforesaid time-series data mainly regarding the apex beats (S15, 16 in FIG. 5), and a waveform resulting from the filtering processing is subjected to absolute value processing and full-wave rectification and is demodulated by finding an envelope curve, similarly to the above (S17 in FIG. 5), and a low-pass filter whose cutoff frequency is 0.15 Hz or lower is further applied to the resultant, whereby it is possible to obtain information of a LF band including a Mayer wave of around 0.1 Hz (cyclic vibration at excitation level of the sympathetic vasoconstrictor nerves), that is, information having an influence on the autonomic nervous system and blood fluctuation (S 18 in FIG. 5).

The inferring means 230 infers biological states by using the aforesaid inference-use processed waveforms obtained by the inference-use processed waveform calculating means 220. Specifically, from the inference processed waveforms, it is possible to infer a respiratory rate, a first-sound interval of heart sound, a heart rate, and so on. Further, the inferring means 230 can be, for example, a means that compares the pseudo-respiratory waveforms reflecting the respiratory physiology information which waveforms are obtained from the time-series data from the three biological signal detection units 11 to 13 and determines which of abdominal breathing and thoracic breathing is predominant or determines the activity state of the respiratory muscles.

Further, the inferring means 230 can be configured to, for example, use two former and latter continuous data regarding the first-sound interval of the pseudo-heart sound waveform, plot the data in sequence on an x-y plane with one of the data (interval (i)) taken on the y coordinate and the other (Interval (i + 1)) taken on the x coordinate to create a Lorenz plot, and evaluate the biological state, for example, heart rate variability, based on a distribution state of points plotted in this Lorenz plot. Similarly, as for the pseudo-waveform of the aortic pulse wave (APW), the pseudo-respiratory waveform, and the Mayer wave as well, a means that creates a Lorenz plot to evaluate the distribution therein can be adopted as the inferring means 230.

### (Experimental examples)

Experiments were conducted in which the above-described biological signal measurement device 1 of the embodiment was disposed on the back of each subject and biological signals (dorsal body surface pulse waves) propagated through the dorsal body surface were captured. At the same time, a finger plethysmogram (PPG) was measured with a finger plethysmogram meter attached to a finger tip of each of the subjects, and an electrocardiogram (ECG) and a phonocardiogram (PCG) were found with sensor parts of an electrocardiograph and a phonocardiograph attached to the chest. Further, breathing was measured with a breathing sensor attached to the abdomen.

In the experiments, whose duration per one time was set to three minutes, the subjects were each requested to first control his/her breathing by active expiration and resting expiration, hold his/her breath for sixty seconds from the start, make effort breathing for 60 to 120 seconds (inhale for five seconds, hold his/her breath for five seconds, and exhale for five seconds), and make natural breathing (free breathing of the subject) for 120 to 180 seconds, and the aforesaid data were measured.

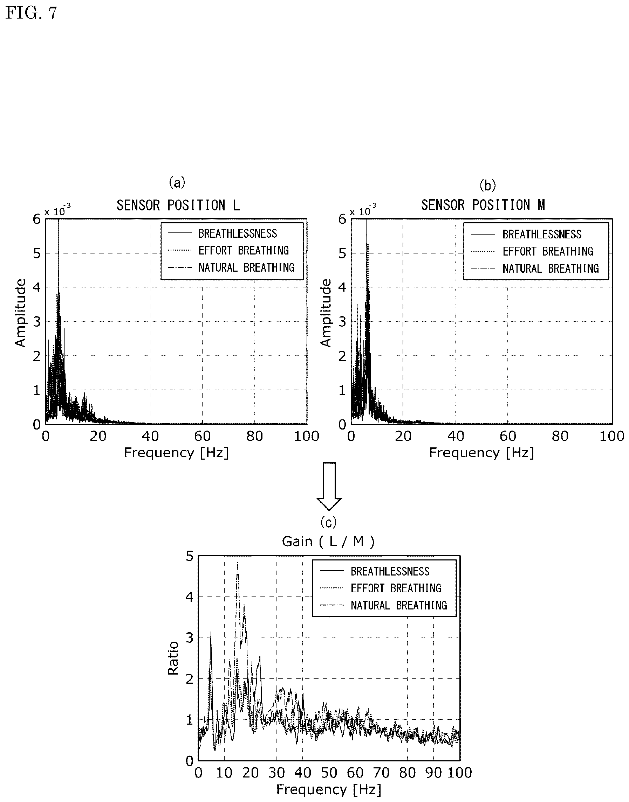

Regarding the subjects H, M, N, FIGs. 6 to FIGs. 8 illustrate the frequency analysis results of time-series data from the acoustic sensor 110 (L) of the left upper part biological signal detection unit 11 of the biological signal measurement device 1, the frequency analysis results of time-series data from the acoustic sensor 110 (M) of the lower part biological signal detection unit 13, and the results of finding a ratio (L/M) of power spectra of these. As is seen in FIG. 6(c), in the data of the subject H, the ratio presents a great change at 35 to 40 Hz and around 50 Hz, and therefore, the frequency deciding means 210 decides that a cutoff frequency of a low-pass filter for use in finding a pseudo-respiratory waveform is, for example, 40 Hz and a pass frequency band of a band-pass filter for use in finding a pseudo-heart sound waveform is 40 to 50 Hz. Similarly, as is seen in FIG. 7(c), in the case of the subject M, the ratio presents a great change at 20 to 30 Hz and around 50 Hz, and therefore, it is decided, for example, that a cutoff frequency of a low-pass filter for use in finding a pseudo-respiratory waveform is 30 Hz and a pass frequency band of a band-pass filter for use in finding a pseudo-heart sound waveform is 30 to 50 Hz. In the case of the subject N in FIG. 8(c), in the same manner, 25 to 30 Hz and around 50 Hz are decided as boundary frequencies for use in filtering.

FIG. 9(a), FIG. 10(a), and FIG. 11(a) are the pseudo-respiratory waveforms of the subjects H, M, N found by the processed waveform calculating means 220. All of the drawings illustrate the pseudo-respiratory waveforms found from the time-series data from the acoustic sensor 110 (L) of the left upper part biological signal detection unit 11, the acoustic sensor 110 (R) of the right upper part biological signal detection unit 12, and the acoustic sensor 110 (M) of the lower part biological signal detection unit 13. Further, for comparison, FIG. 9(b), FIG. 10(b), and FIG. 11(b) illustrate output waveforms of the breathing sensor.

First, from the pseudo-respiratory waveforms, it is seen that there is little amplitude change in the time zone of the breathlessness, an amplitude change is large in the time zone of the effort breathing, and an amplitude change is smaller and its period is shorter in the time zone of the natural breathing than in the time zone of the effort breathing. This is the same tendency as that of the output waveforms of the breathing senor, leading to the understanding that the pseudo-respiratory waveforms accurately reflect the breathing state. Further, the comparison of the three pseudo-respiratory waveforms shows that, in the case of, for example, the subject H, the pseudo-respiratory waveforms corresponding to the acoustic sensor 110 (L) of the left upper part biological signal detection unit 11 and the acoustic sensor 110 (R) of the right upper part biological signal detection unit 12 have larger amplitudes than the pseudo-respiratory waveform corresponding to the acoustic sensor 110 (M) of the lower part biological signal detection unit 13. Therefore, it can be said that the subject H is of a type whose breathing more tends to be thoracic and has well-developed respiratory muscle strength activating the lungs. As for the subject M, the amplitude of the pseudo-respiratory waveform from the acoustic sensor 110 (M) of the lower part biological signal detection unit 13 is larger than the amplitude of the pseudo-respiratory waveform from the acoustic sensor 110 (L) of the left upper part biological signal detection unit 11 and thus it can be said that the breathing of the subject M highly tends to be abdominal. Further, because the amplitude of the pseudo-respiratory waveform from the acoustic sensor 110 (R) of the right upper part biological signal detection unit 12 free from the influence of the movement of the heart is large, the respiratory muscle strength can be evaluated as sufficient. The subject N is of a type whose breathing tends to be thoracic, but since the amplitudes are smaller than those of the pseudo-respiratory waveforms of the subjects H, M, it can be said that an activation amount of the respiratory muscles of the subject N tends to be small as a whole.

The inferring means 230 can be a means that compares the three pseudo-respiratory waveforms as described above and consequently is capable of evaluating the condition (state) of the respiratory physiology of each subject.

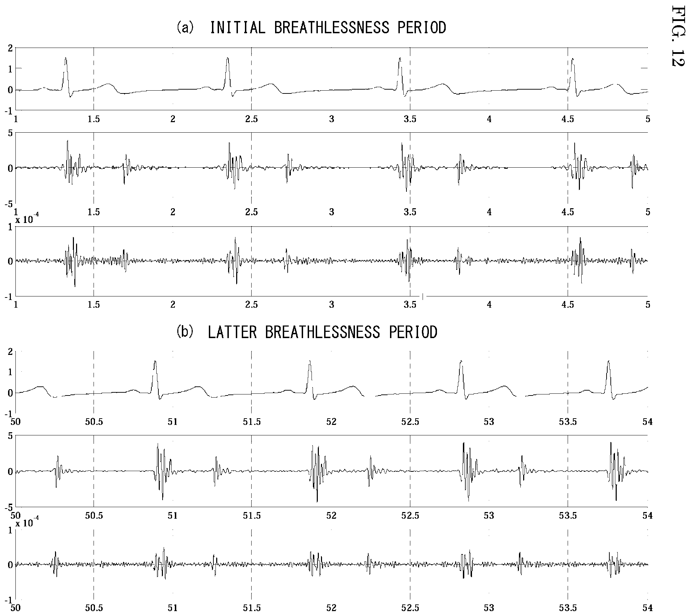

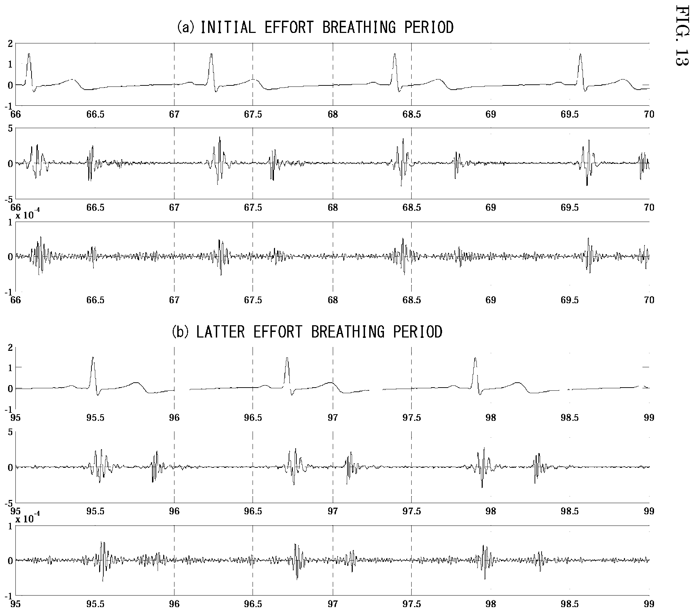

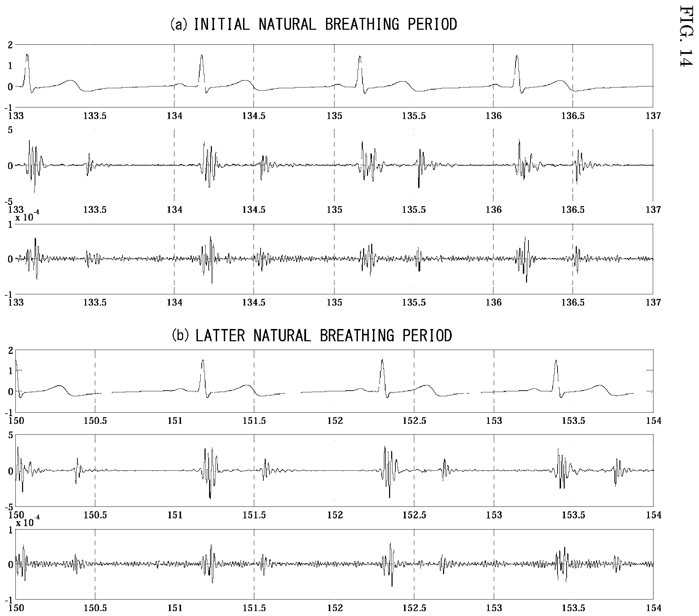

FIGs. 12 to FIGs. 14 illustrate filtered waveforms for pseudo-heart sound waveforms (S7 in FIG. 5) found by the inference-use processed waveform calculating means 220 by applying the aforesaid band-pass filter whose pass frequency band is decided by the filtering frequency deciding means 210 to time-series data of biological signals of the subject N. In more detail, first, time-series waveforms are newly configured by dividing the time-series data obtained from the acoustic sensor 110 (L) of the left upper part biological signal detection unit 11 by those of the acoustic sensor 110 (R) of the right upper part biological signal detection unit 12 and the acoustic sensor 110 (M) of the lower part biological signal detection unit 13, that is, by L/(M × R) (S19 in FIG. 5). Next, a 30 to 50 Hz band-pass filter is applied to these time-series waveforms (S3 in FIG. 5), whereby the filtered waveforms for the pseudo-heart sound waveforms are found (S7 in FIG. 5). In each of FIGs. 12(a), (b), FIGs. 13(a), (b), and FIGs. 14(a), (b), the bottom chart corresponds to the filtered waveform for the pseudo-heart sound waveform at each timing, and data of the phonocardiogram and data of heart sound are also illustrated at the top and the middle respectively. From these drawings, the generation timing of the pseudo-heart sound well agrees with that of the heart sound at any breathing timing.

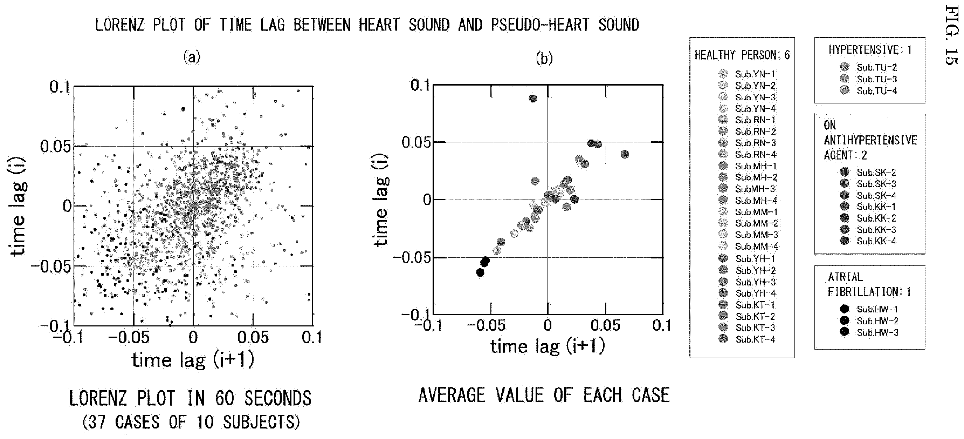

In FIGs. 15, time lags between heart sound data measured from the chest front side by the phonocardiograph and the aforesaid pseudo-heart sound waveform (filtered waveform) are found in a sixty-second period in data of 37 cases of ten subjects. Specifically, using data of two former and latter time lags continuous in time, a Lorenz plot is created by sequentially plotting the data on an x-y plane with one (time lag (i)) of these taken on the y coordinate and the other (time lag (i + 1)) taken on the x coordinate, and evaluation is conducted. FIG. 15(a) illustrates all the plots obtained as a result of analyzing the sixty-second data of the 37 cases, and data within ±0.04 seconds is a normal value. The breakdown of the subjects is: six healthy persons, one hypertensive subject, two subjects on antihypertensive agents, and one subject having developed atrial fibrillation, and because the subjects include many healthy persons, the data of many cases are within ±0.04 seconds. FIG. 15(b) is a chart in which average values taken in each of the 37 cases are plotted. As is seen from this drawing, the data of the healthy persons are within ±0.04 seconds and are substantially all plotted on a line with a 45-degree inclination, but the data of the hypertensive subjects, the subjects on antihypertensive agents, and the subjects having developed atrial fibrillation fall out of the ±0.04 second range and in addition, sometimes presented a tendency of greatly falling out of the 45-degree line. Therefore, the inferring means 230 can infer the biological state, for example, illness, blood pressure, fatigue, and so on by comparing the pseudo-heart sound data with the heart sound data.

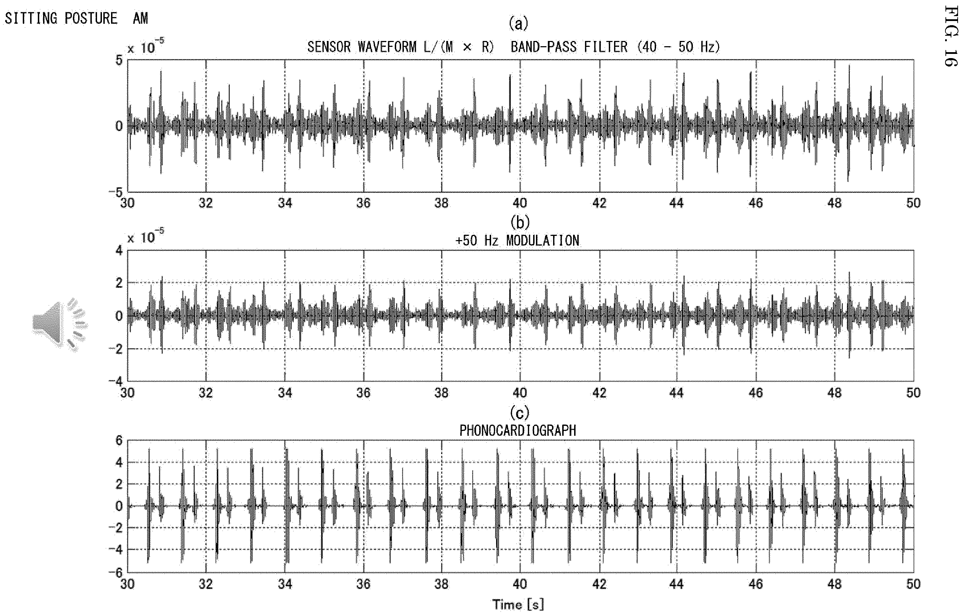

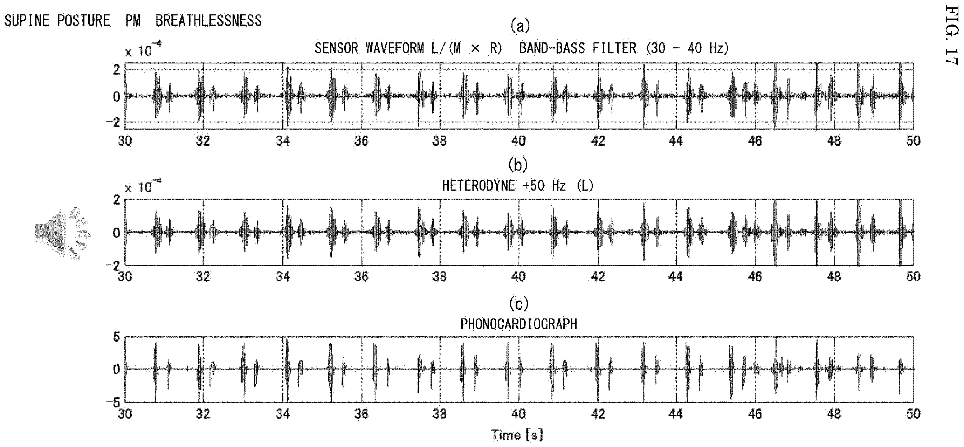

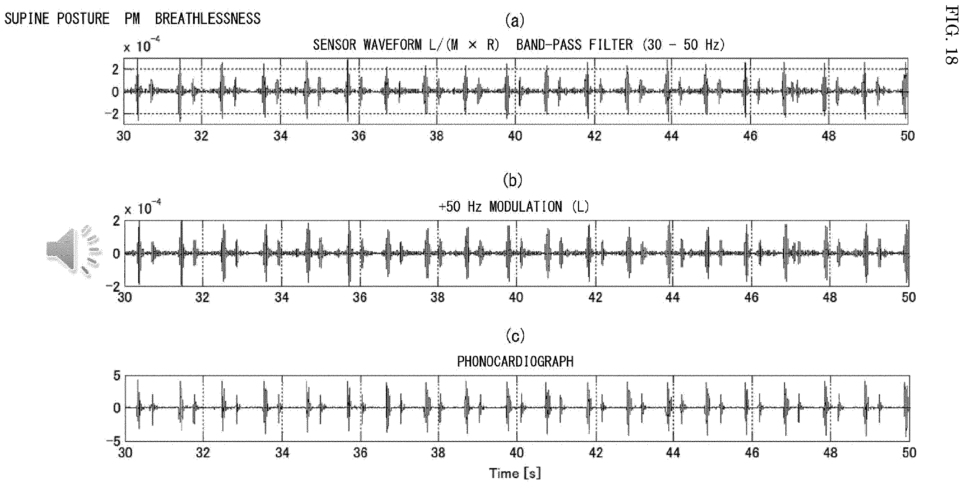

Regarding data of the subjects H, M, N, FIGs. 16 to FIGs. 18 illustrate filtered waveforms for pseudo-heart sound waveforms (waveforms illustrated in FIG. 16(a), FIG. 17(a), FIG. 18(a)) found by applying a band-pass filter (40 to 50 Hz for the subject Hand 30 to 50 Hz for the subject M, N) to time-series waveforms found by L/(M × R) (S19 in FIG. 5), and pseudo-heart sound waveforms (FIG. 16(b), FIG. 17(b), FIG. 18(b)) reproducible to audible sound which waveforms are obtained when the aforesaid filtered waveforms are further subjected to heterodyne processing to be modulated to 70 to 100 Hz (S11 in FIG. 5). These waveforms also well agree with the phonocardiographic waveforms illustrated in FIG. 16(c), FIG. 17(c), and FIG. 18(c).

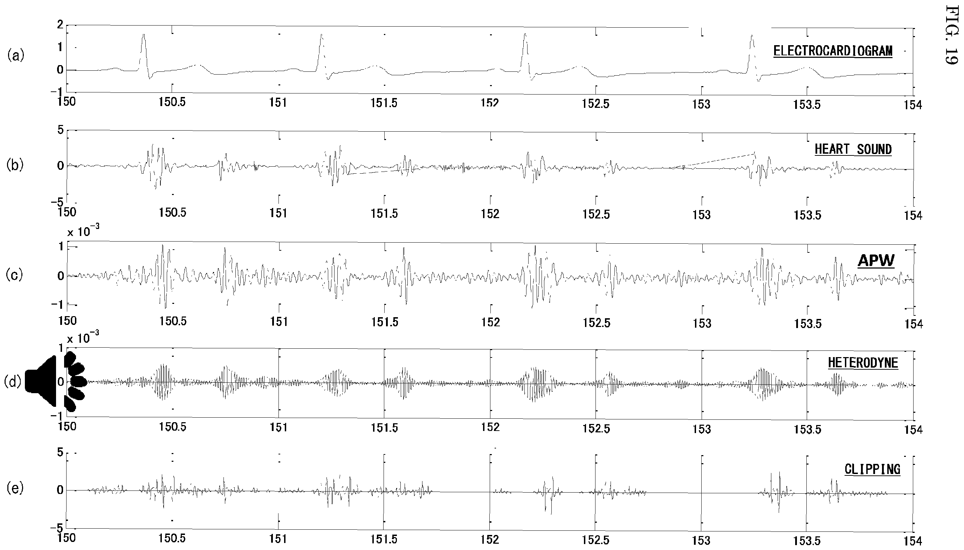

FIGs. 19(a) to (e), which are data of another subject during natural breathing, illustrate an electrocardiogram, a heart sound waveform, a filtered waveform for a pseudo-heart sound waveform (S7 in FIG. 5) resulting from filtering with a band-pass filter (S3 in FIG. 5), a pseudo-heart sound waveform resulting from heterodyne processing (S9, S11 in FIG. 5), and a pseudo-heart sound waveform resulting from clipping processing (S8, S10 in FIG. 5). These drawings also show that the pseudo-heart sound waveforms in FIGs. 19(c) to (e) found from the dorsal body surface pulse waves well agree with the heart sound waveforms.

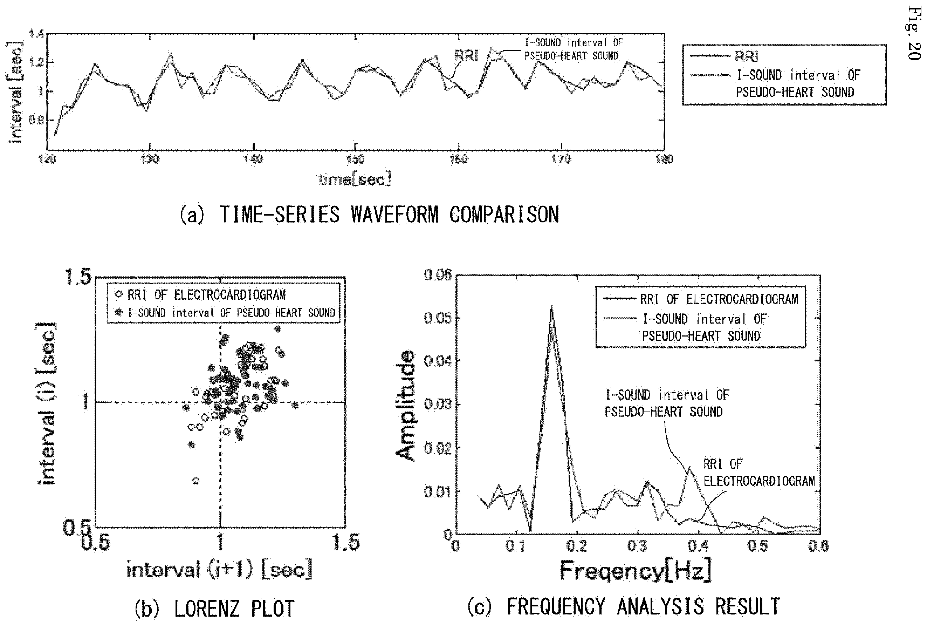

FIGs. 20 illustrate evaluation of deviation between an R wave time interval (RRI) of an electrocardiogram and a first-sound time interval of a pseudo-heart sound waveform (I-sound interval of pseudo-heart sound), (a) illustrating time-series waveforms of these in an overlapping manner, (b) illustrating Lorenz plots of these, and (c) illustrating frequency analysis results of these. From these drawings, it can be said that it is possible to capture heart rate variability from the pseudo-heart sound waveform found from the dorsal body surface pulse wave because period information of the pseudo-heart sound is very similar to period information in the electrocardiogram.

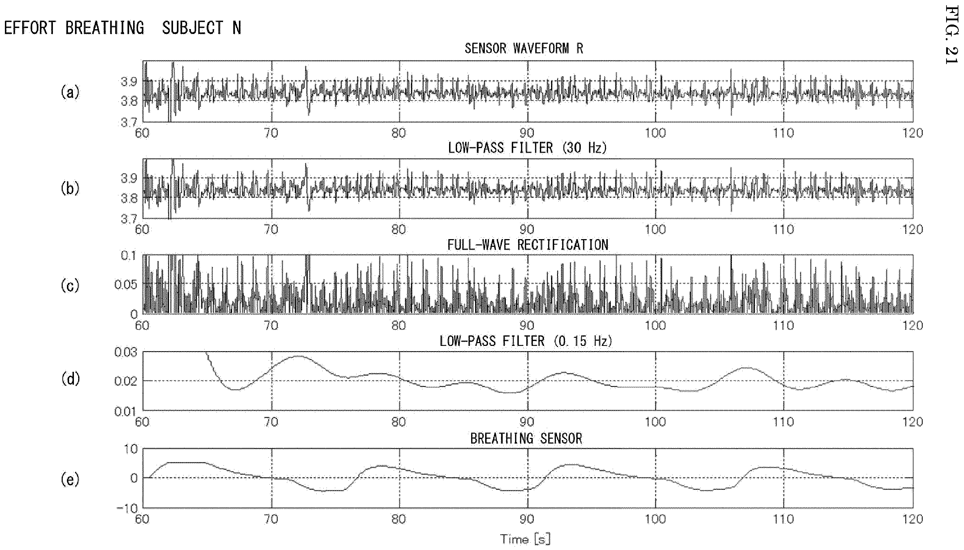

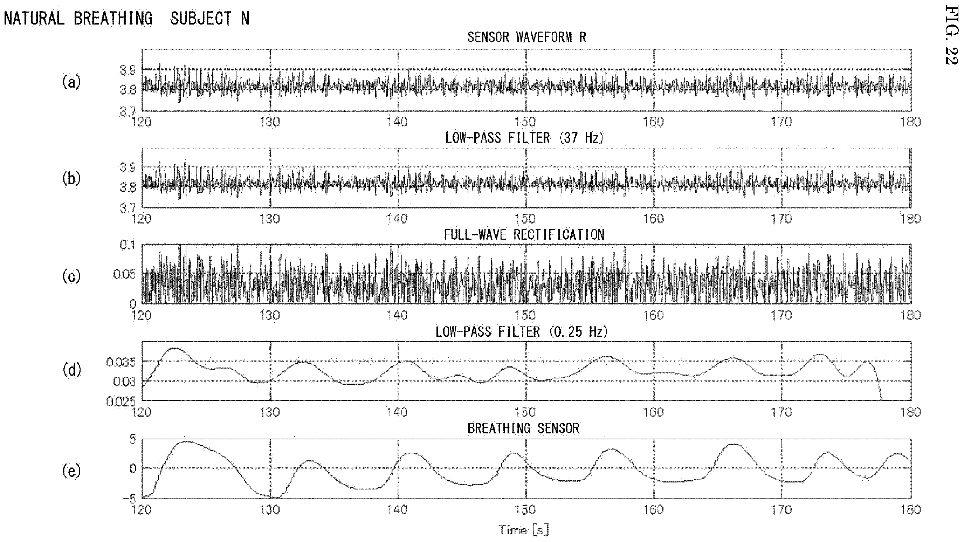

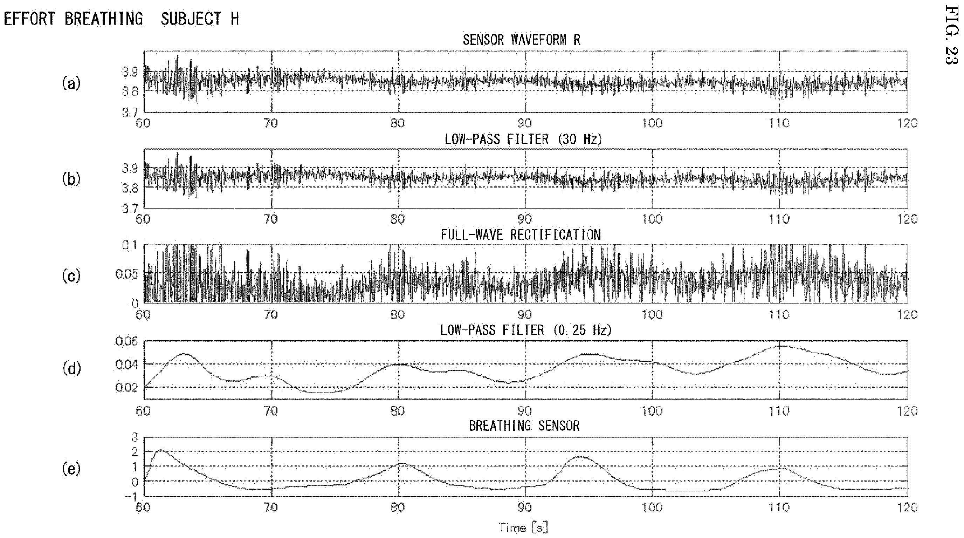

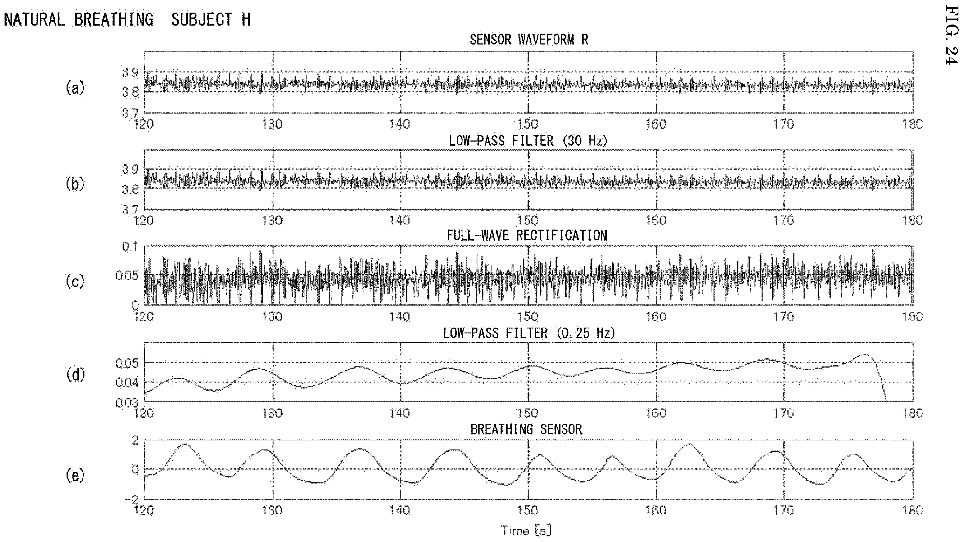

Regarding the time zones of the effort breathing and the natural breathing of the subjects N, H, FIGs. 21 to FIGs. 24 illustrate time-series data (FIG. 21(a), FIG. 22(a), FIG. 23(a), FIG. 24(a)) from the acoustic sensor 110 (R) of the right upper part biological signal detection unit 12, pseudo-respiratory waveforms (filtered waveforms (FIG. 21(b), FIG. 22(b), FIG. 23(b), FIG. 24(b)) created by applying a 30 Hz to 37 Hz low-pass filter to the aforesaid time-series data, waveforms (FIG. 21(c), FIG. 22(c), FIG. 23(c), FIG. 24(c)) created as a result of absolute value processing and full-wave rectification of the aforesaid pseudo-respiratory waveforms, and pseudo-respiratory waveforms (FIG. 21(d), FIG. 22(d), FIG. 23(d), FIG. 24(d)) found by thereafter applying a low-pass filter whose cutoff frequency is 0.15 to 0.25 Hz corresponding to the frequency of breathing to the aforesaid waveforms. It is seen that these waveforms well agree with waveforms of data from the breathing sensor illustrated in FIG. 21(e), FIG. 22(e), FIG. 23(e), and FIG. 24(e).

FIG. 25(a) illustrates, in order from the top, time-series data from the acoustic sensor (M) of the lower part biological signal detection unit 13 in a time zone of natural breathing, a filtered waveform for a pseudo-respiratory waveform found by applying a low-pass filter (30 Hz), a waveform resulting from full-wave rectification, a pseudo-respiratory waveform resulting from filtering with a 0.25 Hz low-pass filter, and a waveform of the breathing sensor. FIG. 25(b) illustrates the frequency analysis result of the pseudo-respiratory waveform, and FIG. 25(c) illustrates the frequency analysis result of the waveform of the breathing sensor. The comparison between the frequency analysis results in FIGs. 25(b), (c) shows that, from the pseudo-respiratory waveform, respiratory physiology information can be detected as is detected by the breathing sensor but it is a waveform including biological information other than the respiratory physiology information.

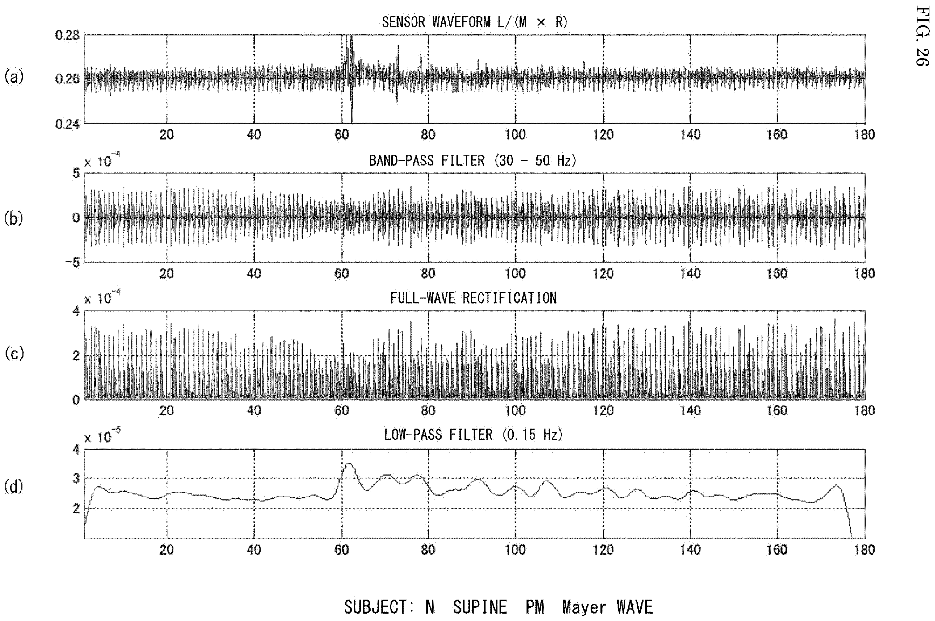

FIGs. 26 are data measured from the subject N in a supine posture, and illustrate a time-series waveform (FIG. 26(a)) found by the aforesaid L/(M × R) (S19 in FIG. 5) using time-series data from the three acoustic sensors 110 in the time zone of the natural breathing, a filtered waveform for a pseudo-heart sound waveform (FIG. 26(b)) found by applying a 30 to 50 Hz band-pass filter to the aforesaid time-series waveform, a waveform (FIG. 26(c)) found through the full-wave rectification of the aforesaid filtered waveform (S16, S17 in FIG. 5), and a time-series waveform (FIG. 26(d)) of a Mayer wave (S18 in FIG. 5) found by further applying a 0.15 Hz low-pass filter to the aforesaid waveform.

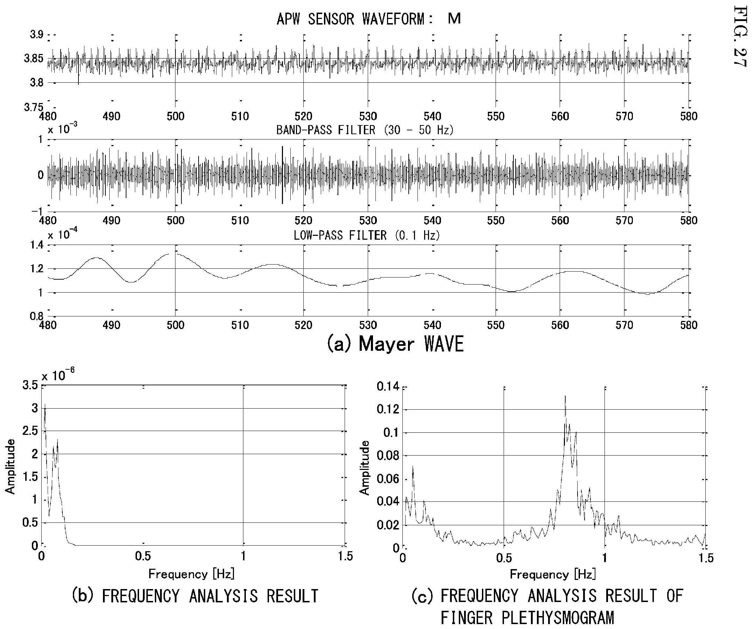

Further, FIGs. 27 illustrate time-series data (the top in FIG. 27(a)) from the acoustic sensor (M) of the lower part biological signal detection unit 13, a filtered waveform for a pseudo-heart sound waveform (the middle in FIG. 27(a)) found by applying a 30 to 50 Hz band-pass filter to the aforesaid time-series data, and a time-series waveform (the bottom in FIG. 27(a)) of a Mayer wave found by further applying a 0.1 Hz low-pass filter to the aforesaid filtered waveform. Note that the data in FIG. 27(a) are the results of an experiment that is conducted with the biological signal measurement device 1 in contact with the back of the subject for ten minutes for the purpose of the clearer acquisition of the Mayer wave. FIG. 27(b) illustrates the frequency analysis result thereof, and FIG. 27(c) illustrates the frequency analysis result of a finger plethysmogram. From FIG. 27(b), it is seen that information of a Lf band including the Mayer wave can be obtained. That is, it is seen that the acoustic sensor (M) of the lower part biological signal detection unit 13 captures peripheral circulatory system information.

### Explanation of Reference Signs

- 1: biological signal measurement device

- 10: base member
- 11: left upper part biological signal detection unit
- 12: right upper part biological signal detection unit
- 13: lower part biological signal detection unit
- 14, 15: film
- 100: three-dimensional knitted fabric
- 110: acoustic sensor
- 20: biological state inference device
- 210: filtering frequency deciding means
- 220: inference-use processed waveform calculating means
- 230: inferring means

## Claims

1. A biological state inference system comprising a biological signal measurement device (1) which is disposed in contact with a back of a person, captures, in a non-constraining manner, a biological signal propagated through a body surface of the back, and configured to transmit time-series data of the biological signal to a biological state inference device (20), the biological signal measurement device (1) comprising:
a left upper part biological signal detection unit (11) which is disposed at a position that is above a diaphragm-corresponding position and on a left side of a backbone-corresponding-position of the person and is configured to obtain time-series data of a biological signal containing central circulatory system information and peripheral circulatory system information that are mainly related to activity of a left cardiac system and respiratory physiology information that is mainly related to activity of a left lung;
a right upper part biological signal detection unit (12) which is disposed at a position that is above the diaphragm-corresponding position and is on a right side of the backbone-corresponding position and is configured to obtain time-series data of a biological signal containing respiratory physiology information mainly related to activity of a right lung; and
a lower part biological signal detection unit (13) which is disposed under the diaphragm-corresponding position and is configured to obtain time-series data of a biological signal containing: abdominal respiratory physiology information mainly related to the activities of the left lung and the right lung and transmitted through a diaphragm; and peripheral circulatory system information, wherein the biological signal measurement device comprises a plate-shaped base member (10) in which detection unit placement holes (10a, 10b, 10c), where to place the left upper part biological signal detection unit (11), the right upper part biological signal detection unit(12), and the lower part biological signal detection unit (13), are formed at three places corresponding to the arrangement positions of the left upper part biological signal detection unit (11), the right upper part biological signal detection unit (12), and the lower part biological signal detection unit (13),
wherein the left upper part biological signal detection unit (11), the right upper part biological signal detection unit (12), and the lower part biological signal detection unit (13) are each composed of a combination of a three-dimensional knitted fabric (100) and an acoustic sensor (110), wherein the biological state inference device (20) is configured to receive the time-series data of the biological signals from the biological signal measurement device (1) , to process the received time-series data of the biological signals to find an inference-use processed waveform for use in inferring a predetermined biological state, and to infer the predetermined biological state from the inference-use processed waveform, the biological state inference device comprising:
a filtering frequency deciding means (210) configured to decide, for each type of the biological state, a filtering frequency for use in finding the inference-use processed waveform, based on frequency analyses of two time-series data or more out of the time-series data of the biological signals from the left upper part biological signal detection unit (11), the right upper part biological signal detection unit (12), and the lower part biological signal detection unit (13);
an inference-use processed waveform calculating means (220) configured to apply the filtering frequency decided for each type of the biological state to the time-series data of the biological signal obtained from at least one of the left upper part biological signal detection unit (11), the right upper part biological signal detection unit (12), and the lower part biological signal detection unit (13) and performs arithmetic processing to find the inference-use processed waveform; and
an inferring means (230) configured to infer the predetermined biological state from the inference-use processed waveform,
**characterized in that**
a dimension along an outer periphery of each of the three-dimensional knitted fabrics is smaller than a dimension along an inner periphery of each of the detection unit placement holes,
the three-dimensional knitted fabrics are supported in the respective detection unit placement holes while pressed by films which are stacked on both surfaces of the base member to cover the detection unit placement holes,
wherein the outer periphery of each of the three-dimensional knitted fabrics is at a predetermined interval from the inner periphery of each of the detection placement holes and the biological state inference device, and
the filtering frequency deciding means (210) is configured to decide the filtering frequency for use in finding the interference-use processed waveform based on a ratio between obtained power spectra corresponding to each frequency in the frequency analyses.

2. The biological state inference system according to claim 1,
wherein the filtering frequency deciding means (210) is a means configured to decide a filtering frequency for respiratory physiology information for use in filtering into time-series data mainly containing respiratory physiology information, by using two frequency analysis results of the time-series data of the biological signal from the left upper part biological signal detection unit (11) and the time-series data of the biological signal from the lower part biological signal detection unit (13), and
wherein the inference-use processed waveform calculating means (220) configured to apply the filtering frequency for respiratory physiology information to the time-series data of the biological signal obtained from at least one of the left upper part biological signal detection unit (11), the right upper part biological signal detection unit (12), and the lower part biological signal detection unit (13) and to perform the arithmetic processing to obtain a pseudo-respiratory waveform as the inference-use processed waveform.

3. The biological state inference system according to claim 2, wherein the inferring means (230) is a means configured to compare data of the two or more pseudo-respiratory waveforms to evaluate activity of a respiratory muscle.

4. The biological state inference system according to claim 1,
wherein the filtering frequency deciding means (210) is a means configured to decide a filtering frequency for heart sound information for use in filtering into time-series data mainly containing heart sound information, by using two frequency analysis results of the time-series data of the biological signal from the left upper part biological signal detection unit (11) and the time-series data of the biological signal from the lower part biological signal detection unit (13), and
wherein the inference-use processed waveform calculating means (220) configured to apply the filtering frequency for heart sound information to the time-series data of the biological signal obtained from at least one of the left upper part biological signal detection unit (11), the right upper part biological signal detection unit (12), and the lower part biological signal detection unit (13) and to perform the arithmetic processing to obtain a pseudo-heart sound waveform as the inference-use processed waveform.

5. The biological state inference system according to claim 4, wherein, after the filtering processing, auralization processing is performed to generate the pseudo-heart sound waveform.

6. The biological state inference system according to claim 5, wherein the auralization processing is clipping processing or heterodyne processing.

7. The biological state inference system according to claim 4, wherein the inferring means (230) includes a means configured to find a time lag between the pseudo-heart sound waveform and heart sound data obtained from a phonocardiograph, to create a Lorenz plot by using the time lag, and to infer the biological state from a variance state in the Lorenz plot.

## Patentansprüche

1. Inferenzsystem für biologische Zustände, das eine Vorrichtung (1) zur Messung biologischer Signale umfasst, die in Kontakt mit einem Rücken einer Person angeordnet ist, in einer nicht einschränkenden Weise ein biologisches Signal erfasst, das sich durch eine Körperoberfläche des Rückens ausbreitet, und ausgebildet ist, um Zeitreihendaten des biologischen Signals an eine Inferenzvorrichtung (20) für biologische Zustände zu übertragen, wobei die Vorrichtung (1) zur Messung biologischer Signale Folgendes umfasst:
eine Erkennungseinheit (11) für biologische Signale für den linken oberen Teil, die an einer Position angeordnet ist, die sich oberhalb einer dem Zwerchfell entsprechenden Position und auf einer linken Seite einer dem Rückgrat entsprechenden Position der Person befindet und so ausgebildet ist, dass sie Zeitreihendaten eines biologischen Signals erhält, das Informationen über das zentrale Kreislaufsystem und Informationen über das periphere Kreislaufsystem enthält, die sich hauptsächlich auf die Aktivität des linken Herzsystems beziehen, und Informationen über Atmungsphysiologie, die sich hauptsächlich auf die Aktivität der linken Lunge beziehen;
eine Erkennungseinheit (12) für biologische Signale für den rechten oberen Teil, die an einer Position angeordnet ist, die sich oberhalb der dem Zwerchfell entsprechenden Position und auf einer rechten Seite der dem Rückgrat entsprechenden Position befindet und so ausgebildet ist, dass sie Zeitreihendaten eines biologischen Signals erhält, die Informationen über Atmungsphysiologie enthalten, die sich hauptsächlich auf die Aktivität einer rechten Lunge beziehen; und
eine Erkennungseinheit (13) für biologische Signale für den unteren Teil, die unter der dem Zwerchfell entsprechenden Position angeordnet ist und so ausgebildet ist, dass sie Zeitreihendaten eines biologischen Signals erhält, enthaltend: Informationen über abdominale Atmungsphysiologie, die sich hauptsächlich auf die Aktivitäten der linken Lunge und der rechten Lunge beziehen und durch ein Zwerchfell übertragen werden; und Informationen über das periphere Kreislaufsystem, wobei die Vorrichtung zur Messung biologischer Signale ein plattenförmiges Basiselement (10) umfasst, in dem Erkennungseinheitsplatzierungslöcher (10a, 10b, 10c), in denen die Erkennungseinheit (11) für biologische Signale für den linken oberen Teil, die Erkennungseinheit (12) für biologische Signale für den rechten oberen Teil und die Erkennungseinheit (13) für biologische Signale für den unteren Teil platziert werden sollen, an drei Stellen gebildet sind, die den Anordnungspositionen der Erkennungseinheit (11) für biologische Signale für den linken oberen Teil, der Erkennungseinheit (12) für biologische Signale für den rechten oberen Teil und der Erkennungseinheit (13) für biologische Signale für den unteren Teil entsprechen,
wobei die Erkennungseinheit (11) für biologische Signale für den linken oberen Teil, die Erkennungseinheit (12) für biologische Signale für den rechten oberen Teil und die Erkennungseinheit (13) für biologische Signale für den unteren Teil jeweils aus einer Kombination aus einem dreidimensionalen Gestrick (100) und einem akustischen Sensor (110) zusammengesetzt sind,
wobei die Inferenzvorrichtung (20) für biologische Zustände so ausgebildet ist, dass sie die Zeitreihendaten der biologischen Signale von der Vorrichtung (1) zur Messung biologischer Signale empfängt, dass sie die empfangenen Zeitreihendaten der biologischen Signale verarbeitet, um eine durch Inferenznutzung verarbeitete Wellenform zur Verwendung bei der Inferenz eines vorbestimmten biologischen Zustands zu finden, und dass sie den vorbestimmten biologischen Zustand aus der durch Inferenznutzung verarbeitete Wellenform folgert, wobei die Inferenzvorrichtung für biologische Zustände Folgendes umfasst:
ein Filterfrequenz-Bestimmungsmittel (210), das so ausgebildet ist, dass es für jeden Typ des biologischen Zustands eine Filterfrequenz zur Verwendung beim Finden der durch Inferenznutzung verarbeiteten Wellenform auf der Grundlage von Frequenzanalysen von zwei Zeitreihendaten oder mehr aus den Zeitreihendaten der biologischen Signale von der Erkennungseinheit (11) für biologische Signale für den linken oberen Teil, der Erkennungseinheit (12) für biologischen Signale für den rechten oberen Teil und der Erkennungseinheit (13) für biologischen Signale für den unteren Teil entscheidet;
ein Mittel zur Berechnung von durch Inferenznutzung verarbeiteten Wellenformen (220), das so ausgebildet ist, dass es die für jeden Typ des biologischen Zustands bestimmte Filterfrequenz auf die Zeitreihendaten des biologischen Signals anwendet, die von mindestens einer von der Erkennungseinheit (11) für biologische Signale für den linken oberen Teil, der Erkennungseinheit (12) für biologische Signale für den rechten oberen Teil und der Erkennungseinheit (13) für biologische Signale für den unteren Teil erhalten wurden, und eine arithmetische Verarbeitung durchführt, um die durch Inferenznutzung verarbeitete Wellenform zu finden; und
ein Inferenzmittel (230), das so ausgebildet ist, dass es den vorbestimmten biologischen Zustand aus der durch Inferenznutzung verarbeiteten Wellenform folgert,
**dadurch gekennzeichnet, dass**
eine Abmessung entlang eines Außenumfangs jedes der dreidimensionalen Gestricke kleiner ist als eine Abmessung entlang eines Innenumfangs jedes der Erkennungseinheitplatzierungslöcher,
die dreidimensionalen Gestricke in den jeweiligen Erkennungseinheitplatzierungslöchern gehalten werden, während sie durch Folien gepresst werden, die auf beiden Oberflächen des Basiselements gestapelt sind, um die Erkennungseinheitplatzierungslöcher abzudecken,
wobei der Außenumfang jedes der dreidimensionalen Gestricke in einem vorbestimmten Abstand vom Innenumfang jedes der Erkennungsplatzierungslöcher und der biologischen Zustandsinferenzvorrichtung liegt, und
das Filterfrequenz-Bestimmungsmittel (210) so ausgebildet ist, dass es die Filterfrequenz zur Verwendung beim Finden der durch Inferenznutzung verarbeiteten Wellenform auf der Grundlage eines Verhältnisses zwischen erhaltenen Leistungsspektren entsprechend jeder Frequenz in den Frequenzanalysen bestimmt.

2. Inferenzsystem für biologische Zustände nach Anspruch 1,
wobei das Filterfrequenz-Bestimmungsmittel (210) ein Mittel ist,
das so ausgebildet ist, dass es eine Filterfrequenz für Informationen über Atmungsphysiologie zur Verwendung beim Filtern in Zeitreihendaten, die hauptsächlich Informationen über Atmungsphysiologie enthalten, unter Verwendung von zwei Frequenzanalyseergebnissen der Zeitreihendaten des biologischen Signals von der Erkennungseinheit (11) für biologische Signale für den linken oberen Teil und der Zeitreihendaten des biologischen Signals von der Erkennungseinheit (13) für biologische Signale für den unteren Teil entscheidet, und
wobei das Mittel zur Berechnung von durch Inferenznutzung verarbeiteten Wellenformen (220) so ausgebildet ist, dass es die Filterfrequenz für die Informationen über Atmungsphysiologie auf die Zeitreihendaten des biologischen Signals anwendet, die von mindestens einer von der Erkennungseinheit (11) für biologische Signale für den linken oberen Teil, der Erkennungseinheit (12) für biologische Signale für den rechten oberen Teil und der Erkennungseinheit (13) für biologische Signale für den unteren Teil erhalten wurden, und dass es die arithmetische Verarbeitung durchführt, um eine pseudo-respiratorische Wellenform als die durch Inferenznutzung verarbeitete Wellenform zu erhalten.

3. Inferenzsystem für biologische Zustände nach Anspruch 2, wobei das Inferenzmittel (230) ein Mittel ist, das so ausgebildet ist, dass es Daten der zwei oder mehr pseudo-respiratorischen Wellenformen vergleicht, um die Aktivität eines Atmungsmuskels zu evaluieren.

4. Inferenzsystem für biologische Zustände nach Anspruch 1,
wobei das Filterfrequenz-Bestimmungsmittel (210) ein Mittel ist, das so ausgebildet ist, dass es eine Filterfrequenz für Herzschallinformationen zur Verwendung beim Filtern in Zeitreihendaten, die hauptsächlich Herzschallinformationen enthalten, unter Verwendung von zwei Frequenzanalyseergebnissen der Zeitreihendaten des biologischen Signals von der Erkennungseinheit (11) für biologische Signale für den linken oberen Teil und der Zeitreihendaten des biologischen Signals von der Erkennungseinheit (13) für biologische Signale für den unteren Teil entscheidet, und
wobei das Mittel zur Berechnung von durch Inferenznutzung verarbeiteten Wellenformen (220) so ausgebildet ist, dass es die Filterfrequenz für Herzschallinformationen auf die Zeitreihendaten des biologischen Signals anwendet, die von mindestens einer von der Erkennungseinheit (11) für biologische Signale für den linken oberen Teil, der Erkennungseinheit (12) für biologische Signale für den rechten oberen Teil und der Erkennungseinheit (13) für biologische Signale für den unteren Teil erhalten wurden; und dass es die arithmetische Verarbeitung durchführt, um eine Pseudo-Herzschall-Wellenform als die durch Inferenznutzung verarbeitete Wellenform zu erhalten.

5. Inferenzsystem für biologische Zustände nach Anspruch 4, wobei nach der Filterverarbeitung eine Auralisationsverarbeitung durchgeführt wird, um die Pseudo-Herzschallwellenform zu erzeugen.

6. Inferenzsystem für biologische Zustände nach Anspruch 5, wobei die Auralisationsverarbeitung eine Clippingverarbeitung oder eine Heterodynverarbeitung ist.

7. Inferenzsystem für biologische Zustände nach Anspruch 4, wobei das Inferenzmittel (230) ein Mittel einschließt, das so ausgebildet ist, dass es eine Zeitverzögerung zwischen der Pseudo-Herzschallwellenform und den von einem Phonokardiographen erhaltenen Herzschalldaten findet, um ein Lorenz-Diagramm unter Verwendung der Zeitverzögerung zu erzeugen und den biologischen Zustand aus einem Varianzzustand im Lorenz-Diagramm zu folgern.

## Revendications

1. Système de déduction d'état biologique comprenant un dispositif de mesure de signal biologique (1) qui est disposé en contact avec le dos d'une personne, qui capture, d'une manière non contraignante, un signal biologique propagé à travers une surface corporelle du dos, et qui est configuré pour transmettre des données de séries temporelles du signal biologique à un dispositif de déduction d'état biologique (20), le dispositif de mesure de signal biologique (1) comprenant :
une unité de détection de signal biologique de partie supérieure gauche (11) qui est disposée à une position qui se situe au-dessus d'une position correspondant au diaphragme et sur un côté gauche d'une position correspondant à la colonne vertébrale de la personne et qui est configurée pour obtenir des données de séries temporelles d'un signal biologique contenant des informations sur le système circulatoire central et des informations sur le système circulatoire périphérique qui sont principalement liées à une activité d'un système cardiaque gauche et des informations de physiologie respiratoire qui sont principalement liées à une activité d'un poumon gauche ;
une unité de détection de signal biologique de partie supérieure droite (12) qui est disposée à une position qui se situe au-dessus de la position correspondant au diaphragme et sur un côté droit de la position correspondant à la colonne vertébrale, et qui est configurée pour obtenir des données de séries temporelles d'un signal biologique contenant des informations de physiologie respiratoire principalement liées à une activité d'un poumon droit ; et
une unité de détection de signal biologique de partie inférieure (13) qui est disposée en-dessous de la position correspondant au diaphragme et qui est configurée pour obtenir des données de séries temporelles d'un signal biologique contenant : des informations de physiologie respiratoire abdominale principalement liées aux activités du poumon gauche et du poumon droit et transmises à travers un diaphragme ; et des informations sur le système circulatoire périphérique, dans lequel le dispositif de mesure de signal biologique comprend un élément de base en forme de plaque (10) dans lequel des trous de placement d'unité de détection (10a, 10b, 10c), où placer l'unité de détection de signal biologique de partie supérieure gauche (11), l'unité de détection de signal biologique de partie supérieure droite (12) et l'unité de détection de signal biologique de partie inférieure (13), sont formés en trois endroits correspondant aux positions d'agencement de l'unité de détection de signal biologique de partie supérieure gauche (11), de l'unité de détection de signal biologique de partie supérieure droite (12) et de l'unité de détection de signal biologique de partie inférieure (13),
dans lequel l'unité de détection de signal biologique de partie supérieure gauche (11), l'unité de détection de signal biologique de partie supérieure droite (12) et l'unité de détection de signal biologique de partie inférieure (13) sont chacune composées d'une combinaison d'un tissu tricoté tridimensionnel (100) et d'un capteur acoustique (110),
dans lequel le dispositif de déduction d'état biologique (20) est configuré pour recevoir les données de séries temporelles des signaux biologiques de la part du dispositif de mesure de signal biologique (1), pour traiter les données de séries temporelles reçues des signaux biologiques afin de trouver une forme d'onde traitée à utilisation déductive à utiliser pour déduire un état biologique prédéterminé, et pour déduire l'état biologique prédéterminé à partir de la forme d'onde traitée à utilisation déductive, le dispositif de déduction d'état biologique comprenant :
un moyen de décision de fréquence de filtrage (210) configuré pour décider, pour chaque type de l'état biologique, d'une fréquence de filtrage à utiliser pour trouver la forme d'onde traitée à utilisation déductive, sur la base d'analyses de fréquence de deux données de séries temporelles ou plus parmi les données de séries temporelles des signaux biologiques provenant de l'unité de détection de signal biologique de partie supérieure gauche (11), de l'unité de détection de signal biologique de partie supérieure droite (12), et de l'unité de détection de signal biologique de partie inférieure (13) ;
un moyen de calcul de forme d'onde traitée à utilisation déductive (220) configuré pour appliquer la fréquence de filtrage décidée pour chaque type de l'état biologique aux données de séries temporelles du signal biologique obtenues à partir d'au moins une parmi l'unité de détection de signal biologique de partie supérieure gauche (11), l'unité de détection de signal biologique de partie supérieure droite (12) et l'unité de détection de signal biologique de partie inférieure (13) et qui met en oeuvre un traitement arithmétique pour trouver la forme d'onde traitée à utilisation déductive ; et
un moyen de déduction (230) configuré pour déduire l'état biologique prédéterminé à partir de la forme d'onde traitée à utilisation déductive,
**caractérisé en ce que**
une dimension le long d'une périphérie externe de chacun des tissus tricotés tridimensionnels est inférieure à une dimension le long d'une périphérie interne de chacun des trous de placement d'unité de détection,
les tissus tricotés tridimensionnels sont supportés dans les trous de placement d'unité de détection respectifs tout en étant pressés par des films qui sont empilés sur les deux surfaces de l'élément de base afin de recouvrir les trous de placement d'unité de détection,
dans lequel la périphérie externe de chacun des tissus tricotés tridimensionnels est à un intervalle prédéterminé de la périphérie interne de chacun des trous de placement de détection et du dispositif de déduction d'état biologique, et
le moyen de décision de fréquence de filtrage (210) est configuré pour décider de la fréquence de filtrage à utiliser pour trouver la forme d'onde traitée à utilisation déductive sur la base d'un rapport entre des spectres de puissance obtenus correspondant à chaque fréquence dans les analyses de fréquence.

2. Système de déduction d'état biologique selon la revendication 1,
dans lequel le moyen de décision de fréquence de filtrage (210) est un moyen configuré pour décider d'une fréquence de filtrage pour les informations de physiologie respiratoire à utiliser pour filtrer les données de séries temporelles contenant principalement des informations de physiologie respiratoire, en utilisant deux résultats d'analyse de fréquence des données de séries temporelles du signal biologique provenant de l'unité de détection de signal biologique de partie supérieure gauche (11) et des données de séries temporelles du signal biologique provenant de l'unité de détection de signal biologique de partie inférieure (13), et
dans lequel le moyen de calcul de forme d'onde traitée à utilisation déductive (220) configuré pour appliquer la fréquence de filtrage pour les informations de physiologie respiratoire aux données de séries temporelles du signal biologique obtenues à partir d'au moins une parmi l'unité de détection de signal biologique de partie supérieure gauche (11), l'unité de détection de signal biologique de partie supérieure droite (12) et l'unité de détection de signal biologique de partie inférieure (13) et pour mettre en oeuvre le traitement arithmétique afin d'obtenir une forme d'onde pseudo-respiratoire comme forme d'onde traitée à utilisation déductive.

3. Système de déduction d'état biologique selon la revendication 2, dans lequel le moyen de déduction (230) est un moyen configuré pour comparer des données des deux ou plus de deux formes d'ondes pseudo-respiratoires afin d'évaluer une activité d'un muscle respiratoire.

4. Système de déduction d'état biologique selon la revendication 1,
dans lequel le moyen de décision de fréquence de filtrage (210) est un moyen configuré pour décider d'une fréquence de filtrage pour des informations sonores cardiaques à utiliser pour filtrer les données de séries temporelles contenant principalement des informations sonores cardiaques, en utilisant deux résultats d'analyse de fréquence des données de séries temporelles du signal biologique provenant de l'unité de détection de signal biologique de partie supérieure gauche (11) et des données de séries temporelles du signal biologique provenant de l'unité de détection de signal biologique de partie inférieure (13), et
dans lequel le moyen de calcul de forme d'onde traitée à utilisation déductive (220) configuré pour appliquer la fréquence de filtrage pour les informations sonores cardiaques aux données de séries temporelles du signal biologique obtenues à partir d'au moins une parmi l'unité de détection de signal biologique de partie supérieure gauche (11), l'unité de détection de signal biologique de partie supérieure droite (12), et l'unité de détection de signal biologique de partie inférieure (13) et pour mettre en oeuvre le traitement arithmétique pour obtenir une forme d'onde sonore pseudo-cardiaque comme forme d'onde traitée à utilisation déductive.

5. Système de déduction d'état biologique selon la revendication 4, dans lequel, après le traitement de filtrage, un traitement d'auralisation est mis en oeuvre pour générer la forme d'onde sonore pseudo-cardiaque.

6. Système de déduction d'état biologique selon la revendication 5, dans lequel le traitement d'auralisation est un traitement d'écrêtage ou un traitement hétérodyne.

7. Système de déduction d'état biologique selon la revendication 4, dans lequel le moyen de déduction (230) inclut un moyen configuré pour trouver un décalage temporel entre la forme d'onde sonore pseudo-cardiaque et les données sonores cardiaques obtenues à partir d'un phonocardiographe, afin de créer une courbe de Lorenz en utilisant le décalage temporel, et pour déduire l'état biologique à partir d'un état de variance dans la courbe de Lorenz.
